# EUROPEAN PATENT SPECIFICATION

(11) **EP 3 174 528 B1**
(45) Date of publication and mention of the grant of the patent: **01.07.2026**
(21) Application number: 15756442.8
(22) Date of filing: 31.07.2015
(51) Int. Cl.: A61P 35/00, A61K 9/14, A61K 9/16, A61P 43/00

(54) **A METHOD OF PREPARING AMORPHOUS SOLID DISPERSION IN SUBMICRON RANGE BY CO-PRECIPITATION**
VERFAHREN ZUR HERSTELLUNG EINER AMORPHEN FESTEN DISPERSION IM SUBMIKRONBEREICH DURCH KOPRÄZIPITATION
PROCÉDÉ DE PRÉPARATION D'UNE DISPERSION SOLIDE AMORPHE DANS LE DOMAINE SUBMICRONIQUE PAR COPRÉCIPITATION

(30) Priority: 01.08.2014 PT 10784614
(43) Date of publication of application: 07.06.2017
(73) Proprietor: Hovione International Ltd., Hong Kong (HK)
(72) Inventor: TEMTEM, Marcio, P-2975-329 Quinta do Conde (PT); PEREIRA, Ruben, P-1150-046 Lisboa (PT); VICENTE, João, 1B Lisboa 1990-136 (PT); GASPER, Filipe, P-2780-308 Oeiras (PT); DUARTE, Iris, P-1600-371 Lisboa (PT)
(74) Representative: Paulraj, Leonita Theresa
(86) International application number: PCT/GB2015/052233
(87) International publication number: WO 2016/016665

(56) References cited:
- EP-A2- 0 988 863
- WO-A1-2013/105894
- CN-Y- 201 337 903
- US-A1- 2009 269 250
- ZHU W Z ET AL: "Liquid antisolvent preparation of amorphous cefuroxime axetil nanoparticles in a tube-in-tube microchannel reactor", INTERNATIONAL JOURNAL OF PHARMACEUTICS, ELSEVIER BV, NL, vol. 395, no. 1-2, 16 August 2010 (2010-08-16), pages 260 - 265, XP027122855, ISSN: 0378-5173, [retrieved on 20100521]

## Description

### 1. Field of invention

The present invention relates to a method of producing amorphous solid dispersions by performing solvent controlled co-precipitation in an apparatus that facilitates molecular contact/interaction within a defined reaction chamber or micro channel, hereinafter, called microreaction technology (MRT). Particularly, the present invention relates to a method of producing amorphous solid dispersions of pharmaceutically active compounds (APIs) in a particulate form, with particle size in the submicron range, amorphous solid dispersions obtained by the method and their uses. The method can be applied in the pharmaceutical field particularly in the processing of active pharmaceutical ingredients, intermediate drug products or drug products. The process described herein is compatible with the continuous manufacturing, and allows the synthesis of solid dispersions and particle engineering in a single step. Moreover, the amorphous particulates produced in accordance with the method of present invention present advantageous characteristics in terms of particle size and density.

### 2 Background of the invention

Up to 90% of the active pharmaceutical substances under development are poorly water soluble, usually resulting in low bioavailability. To overcome this, and promote the successful translation of novel chemical entities into pharmaceutical drug products, different engineering and formulation approaches have been developed: particle design and size reduction techniques, self-emulsifying drug delivery systems, cyclodextrin complexes, amorphous solid dispersions, salt forms and cocrystals forms. Among the different alternatives, the use of stabilized amorphous solid dispersions is becoming an increasingly popular platform with a high number of drugs reaching the market. An amorphous solid dispersion comprises at least two components, generally a stabilizing agent (eg. a polymer) and a drug. The distinctive advantage of amorphous solid dispersions compared with other formulation strategies is that, once the drug starts to dissolve in the site of absorption, a supersaturation state is obtained, i.e. the concentration of the drug reaches values well above its intrinsic solubility. Bioavailability enhancement may be achieved by improving the dissolution kinetics (applicable to Class Ila compounds, according to the Developability Classification System, DCS or the Biopharmaceutics Classification System, BCS) and/or by increasing the maximum concentration of the active compound in solution (applicable to DCS Class Ilb compounds). In the cases where dissolution kinetics is key to achieve bioavailability, the properties of the amorphous solid dispersions, namely particle size, can play an important role in the dissolution profile of the drug product.

Although various methods are reported in literature for the preparation of solid dispersions (e.g. spray drying, freeze drying, hot melt extrusion) the state of the art is scarce in technologies that enable both control of the particle size in the submicron range while maintaining the amorphous nature of the solid dispersion particulates. Spray dried particles are typically hollow, with a low density and particle size in the range of 2-120 microns, in opposition extrudates from hot melt extrusion are dense, with very coarse particles or pellets and requiring additional downstream processing (e.g. milling) to obtain fine material.

One of the objectives of the present invention is to provide an alternative co-precipitation process that uses microreaction or microfluidization to promote molecular contact or interaction between the streams comprising the active ingredients, excipients such as stabilizing agents, and solvent/anit-solvent systems, and to obtain amorphous solid dispersions in the submicron range with high density.

In the field of application of inorganic compounds, Filipa Castro et al. (PhD thesis, Process Intensification for the Production of Hydroxyapatite Nanoparticles, Univ. Minho, 2013) microreaction technology was applied to improve the production and characteristics of hydroxyapatite nanoparticles. When compared with traditional approaches, like stirred vessels, Filipa Castro et al. observed advantages due to the increase in the surface to volume ratio enhancing heat and mass transfer.

In the field of application of pharmaceutical compounds, the state-of-the-art includes a few examples of similar approaches in the processing of drug-alone particles and/or in the processing of crystalline materials. US 8,367,004 B2 discloses a method to produce crystals or polymorphs with particle size in the submicron range. Hany Ali et al. (Iranian Journal of Pharmaceutical Research, 13 (3), 2014, 785-795) describes a bottom-up technique to produce nano-crystals of budesonide. Hong Zhao et al. (Ind. Eng. Chem. Res., 46 (24), 2007, 8229-8235) described a method to produce drug-alone crystalline particles of an active pharmaceutical ingredient in the sub-microns region. Chinese Patent CN201337903YA also disclosed details of a new setup for the production of an amorphous drug-alone particulate product, cefuroxime axetil. Although the abovementioned references provide a deep insight of the microfluidization or microreaction and its benefits, surprisingly the co-precipitation of amorphous solid dispersions was never assessed in the state of the art.

The term "microreaction" refers to a technology that involves physical and/or chemical reactions within microreactors, micromixers, microchannels or any other component comprised within the microfluidic field.

The term "microfluidization" refers to microfluidic reaction technology (MRT), which encompasses both hardware such as apparatus and processes. The MRT may be used to produce nanoparticles and/or expedite the rate of chemical reactions by minimizing diffusion limitations between single-phase and multiphase reactant streams. The technology involves high shear, continuous fluid processing through a fixed geometry which provides intense and uniform mixing in the meso- and micromixing range and generates nanometer scale eddies and products.

The term "amorphous solid dispersion" is defined as the dispersion of at least one drug in a matrix, in the amorphous state. The matrix may comprise polymers, surfactants or mixtures thereof. In the scope of this invention this term is also used to describe co-precipitates, in the form of amorphous nanoparticles containing both the active ingredient and the matrix.

In the case of pharmaceutical amorphous solid dispersions, the selection of the ingredients, the solvent and/or anti-solvent system, the individual concentrations of each component and the mixing conditions are crucial for the simultaneous precipitation or co-precipitation of all the constituents, in such a way that the composition of the precipitated particles corresponds to the intended formulation. The method herein disclosed addresses not only the challenge of production of pharmaceutical amorphous solid dispersions but also the control of such particulate system in the submicron range. Thus, it is appropriate to tackle both dissolution rate and/or solubility limited pharmaceutical compounds, commonly designated by BCS class II compounds.

In the field of the production of submicron particles through solvent controlled co-precipitation the state-of-the-art also includes a number of meaningful examples. US 7,0375,28 B2 discloses a process where co-precipitated particles are obtained through solvent controlled precipitation using acidified cold water as anti-solvent and enteric polymers as dispersing agents. Following the co-precipitation step, the patent describes the execution of a high energy step in order to obtain particle sizes ranging from 0.4 µm to 2.0 µm. However, producing co-precipitated particles through this method does not allow the control over the solid state of the materials, which can be amorphous, crystalline or semi-crystalline. Moreover, the use of a third step following the co-precipitation step indicates the particle size is stabilized by subjecting particles to high energy conditions. Another limitation of US 7,037,528 B2 refers to the obligatory use of surfactants in the formulation. The present invention addresses all these limitations.

WO 2013105894A1 describes a manufacturing method to produce amorphous hybrid nanoparticles by mixing two streams and spraying the mixture through a nozzle and drying. This method uses a supercritical fluid in one of the streams in order to precipitate the material, prior to atomization, and collects the particles dried from the spray. Although this method is suitable to produce amorphous solid dispersions with particle size in the submicron range, it is limited by the solubility of the compounds in the supercritical fluid, typically carbon dioxide, and adds the challenges of processing feeds with gases at high pressures and temperatures with a supercritical fluid incorporated that is a serious hurdle for commercial manufacturing.

Hence, the inventors of the present invention have appreciated the need for providing a method of producing amorphous solid dispersions, particularly in the submicron range. This is achieved by the method of the present invention, which enables the production of pharmaceutical amorphous solid dispersed particles in a single manufacturing step with a stable size down to at least 50 nm. The method uses microreaction or microfluidization to promote molecular contact or interaction between the streams comprising the active ingredients to obtain amorphous solid dispersions in the submicron range with high density. Also, the technology is adaptable to continuous processing and is easily scalable to commercial scales. Furthermore, the solubility limitation of the ingredients is minimized as they can be dissolved either in the solvent system and/or anti-solvent system.

### 3. Summary of the invention

According to one aspect of the present invention there is provided a method of manufacturing amorphous solid dispersions in a particulate form, which method comprises: (i) preparing a solution comprising at least one pharmaceutically active compound and a solution comprising at least one stabilizing agent, wherein each solution is prepared using a first solvent, and (ii) mixing the solutions with a second solvent which comprises at least one anti-solvent by means of microfluidization or a microreaction to obtain a suspension of amorphous particles by co-precipitation, wherein the stabilizing agent is at least one polymer selected from the group comprising: cellulose ester, cellulose ether, polyalkylene oxide, polyacrylate, polymethacrylate, polyacrylamide, polyvinyl alcohol, vinyl acetate polymer, oligosaccharide, polysaccharide, hydroxypropylcellulose, polyvinylpyrrolidone, hydroxyalkylcelluloses, hydroxyalkylalkylcellulose, hydroxypropylmethylcellulose, cellulose phthalate, cellulose succinate, cellulose acetate phthalate, hydroxypropylmethylcellulose phthalate, hydroxypropylmethylcellulose acetate succinate, polyethylene oxide, polypropylene oxide, copolymer of ethylene oxide and propylene oxide, methacrylic acid/ethyl acrylate copolymer, methacrylic acid/methyl methacrylate copolymer, hydroxypropylmethylcellulose succinate, butyl methacrylate/2-dimethylaminoethyl methacrylate copolymer, poly(hydroxyalkyl acrylate), poly(hydroxyalkyl methacrylate), gelatin, copolymer of vinyl acetate and crotonic acid, partially hydrolyzed polyvinyl acetate, carrageenan, galactomannan, high viscosity gums or xanthan gum, and a combination thereof, and/or at least one surfactant which comprises an anionic surfactant, a cationic surfactant, a nonionic surfactant or a combination thereof.

Preferably, the solution comprising at least one pharmaceutically active compound and the solution comprising at least one stabilizing agent are combined to form a first stream, prior to mixing with the second solvent, which may be an anti-solvent of both the pharmaceutically active ingredient and the stabilizing agent. Preferably, the solution comprising the stabilizing agent is combined with the second solvent to form a second stream, which second stream comprises an anti-solvent of the pharmaceutically active compound.

Also disclosed is a method of manufacturing amorphous solid dispersions in a particulate form, which method comprises: (i) preparing a solution comprising at least one pharmaceutically active compound using a first solvent and a solution comprising at least one stabilizing agent using a second solvent; wherein the second solvent is an anti-solvent of the pharmaceutically active compound; and (ii) mixing the solutions by means of microfluidization or a microreaction to obtain a suspension of amorphous particles by co-precipitation.

The method preferably comprises an isolation step to separate the amorphous particles in the form of a powder.

Also disclosed is a particulate amorphous solid dispersion obtained by the method of the present invention, the dispersion comprising 5 to 95 % (w/w) of the pharmaceutically active compound and 95 to 5 % (w/w) of the stabilizing agent. The stabilizing agents are, at least one surfactant and/or polymer.

Also disclosed is a pharmaceutical composition comprising a particulate amorphous solid dispersion as described herein.

Also disclosed is a pharmaceutical composition comprising a particulate amorphous solid dispersion for use as a medicament.

Also disclosed is a particulate amorphous solid dispersion for use in increasing the bioavailability of a pharmaceutically active compound.

The foregoing and other features and advantages of the invention will be more readily understood upon consideration of the following detailed description of the invention, and the accompanying drawings.

### 4. Brief description of the drawings

These drawings illustrate certain aspects of some of the embodiments of the present invention, and should not be used to limit or define the invention.
Figure 1 shows a schematic representation of the process of the invention.
Figure 2 shows the XRPD patterns correspondent to the 10 wt.% and 40 wt.% drug load ltraconazole:Eudragit^{®} L100 co-precipitates (A1 and A2 spectra, respectively).
Figure 3 shows the XRPD patterns correspondent to the 10 wt.% Cinnarizine: Eudragit^{®} L100 co-precipitates, isolated via filtration plus drying in a tray drier oven (B1) and isolated via spray drying (B2).
Figure 4 shows SEM micrographs correspondent to the 10 wt.% Cinnarizine : Eudragit^{®} L100 co-precipitated product isolated via spray drying at a) 1000x, b) 3000x, c) 10,000x and b) 30,000x magnification.
Figure 5 shows the XRPD patterns correspondent to the 10 wt.% and 40 wt.% drug load Nilotinib: Eudragit^{®} L100 co-precipitates (C1 and C2 spectra, respectively).
Figure 6 shows the powder dissolution profiles, obtained over 240 min, of the 40 wt.% Nilotinib:Eudragit^{®} L100 NanoAmorphous formulation (A), 40 wt.% Nilotinib:Eudragit^{®} L100 MicroAmorphous formulation (B) and Nilotinib in the crystalline state (C).
Figure 7 shows XRPD difractograms correspondent to the 20 wt.% Carbamazepine:Eudragit^{®} L100 after co-precipitation (A) and after isolation by spray drying (B).
Figure 8 shows SEM micrographs correspondent to the 20 wt.% Carbamazepine:Eudragit^{®} L100 co-precipitated product at a)1500x, b) 3000x, c) 10,000x and d) 40,000x magnification.
Figure 9 shows SEM micrographs correspondent to 20 wt.% Carbamazepine:Eudragit^{®} L100 spray dried product at a) 500x, b) 1000x, c) 5,000x and d) 20,000x magnification.
Figure 10 shows the powder in-vitro dissolution profiles for the materials produced under the scope of this invention (NanoAmorphous - A) and by spray drying (MicroAmorphous - B).
Figure 11 shows Pharmacokinetic profiles, obtained over 180 min, for a formulation with 20 wt.% Carbamazepine:Eudragit^{®} L100 NanoAmorphous (A), MicroAmorphous (B) and model Carbamazepine in the crystalline state (C).

### 5. Detailed Description of the invention

The present invention provides a method for producing amorphous solid dispersions in a particulate form comprising at least one pharmaceutically active compound and at least one stabilizing agent, with particle size in the submicron range.

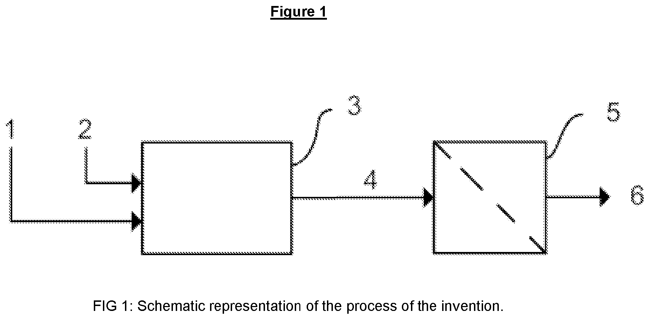

The manufacturing process, as shown in Figure 1, may be divided into three stages:
Stage (i) - two solvents (the first solvent and the second solvent) are selected. The solvents are selected such that the active pharmaceutical compound of interest is partially soluble in one solvent, hereinafter referred just as "the solvent" and substantially insoluble in the other solvent, hereinafter referred just as "the anti-solvent". In a preferred aspect, both the pharmaceutically active compound(s) and the stabilizing agent (i.e. polymer(s) and/or surfactants(s)) are dissolved or partially dissolved in the first solvent and the second solvent comprises an anti-solvent of both the pharmaceutically active compound(s) and the stabilizing agent. In a preferred aspect, the pharmaceutically active compound(s) is dissolved or partially dissolved in the first solvent and the second solvent is an anti-solvent of the pharmaceutically active compound(s). In a preferred aspect, the stabilizing agent (i.e. surfactants, polymers) is dissolved or partially dissolved in the second solvent, the second solvent being an anti-solvent of the pharmaceutically active compound(s). The term "anti-solvent" is used herein to describe a solvent that said substance/compound(s) shows a substantially lower solubility in. When more than one substance/compound(s) is mixed with the common anti-solvent, the substances precipitate within the anti-solvent as opposed to dissolving within in, preferably forming composite particles made of the different substances.

Preferably, the term "anti-solvent" is used herein to describe a solvent or a mixture of solvents wherein said substance shows a substantially lower solubility when compared with the solvent. Preferably, the term "anti-solvent" is used to refer to a solvent in which said substance is completely insoluble.

Preferably, the term "solvent" is used herein to describe a solvent or a mixture of solvents wherein said substance shows solubility in the proportions of up to 50 volumes/g of solute. The term "volumes/g" is a measure that refers to milliliters of solvent per gram of solute.

The person skilled in the art would be able to select a solvent that may be used as a solvent for a particular compound and as an anti-solvent for another compound.

Preferably, the term "soluble" means from 10 to 30 parts solvent is needed to dissolve 1 part solute.

Preferably, the term "substantially lower solubility" means from 100 to 1000 parts solvent is needed to dissolve 1 part solute.

Preferably, the term "substantially insoluble" means from 1000 to 10,000 parts solvent is needed to dissolve 1 part solute; and the term "insoluble" means more than10,000 parts solvent is needed to dissolve 1 part solute.

The terms 'solvent' part(s) and 'solute' part(s) refer to appropriate volume of solvent in milliliters per gram of solute.

In stage (i) a first stream (1) comprising a solution of one or more pharmaceutically active compounds and one or more stabilizing agents (i.e. polymer(s) and/or surfactants(s)), which are capable of forming co-precipitates in at least one solvent/anti-solvent system is prepared. Preferably, a solution of one or more pharmaceutically active compounds and a solution of one or more stabilizing agents (i.e. polymer(s) and/or surfactants(s)) are prepared separately and then combined to form the first stream. A second stream (2) comprising a second solvent which comprises an anti-solvent of the pharmaceutically active compounds and the stabilizing agent is prepared.

In one preferred aspect, the solution of one or more stabilizing agents is combined with the second solvent to form the second stream, or the stabilizing agent may be added directly to the second solvent to form the second stream. In this case, the second solvent may act as a solvent of the stabilizing agent, and as an anti-solvent of the pharmaceutically active compound (s) present in the first stream.

Preferably, the ratio of the pharmaceutically active compound to the stabilizing agent present in the solution(s) is in the range of from about 95 to 5 (% w/w) to about 5 to 95 (% w/w).

Stage (ii) - The first stream (1) and the second stream (2) are mixed under controlled conditions in an apparatus (3) for effecting microfluidization or microreaction. The apparatus for effecting microfluidization is, preferably, a microreactor or a microfluidics reaction technology (MRT) device, or any similar devices that facilitates highly effective molecular contact/interaction within a defined reaction chamber or micro channels, to form a suspension (4) of amorphous nanoparticles by co-precipitation of the substances in the two streams. Preferably, the reaction chamber comprises one or more channels of well-defined diameter and size. Preferably, the diameter of the channels is in the range of about 10 microns to about 400 microns. More preferably, the diameter is in the range of about 50 microns to about 200 microns. One or more apparatus, for example microreactor(s) or MRTs may be used in series or in parallel. The process that is carried in the apparatus is preferably a continuous process.

Preferably, the solutions are continuously pumped into the reaction chamber where they are mixed and allowed to react (continuous flow reaction).

The first and second streams are preferably fed to one or more intensifier pumps at different individually controlled rates such that interaction between the first and second streams is substantially controlled prior to feeding the streams to the apparatus (3) for microfluidization or microreaction. Preferably, the overall flow rate i.e. the flow rate of the two streams comprising the active substance(s), excipients (stabilizing agent), solvent and anti-solvent is controlled by using at least one intensifier pump. The overall flow rate may be up to 50 kg/h.

Preferably, a peristaltic pump may be used for controlling the flow rate of at least one of the two streams i.e. the solvent and anti-solvent streams. Flow rates of both the streams may be controlled individually and therefore, the flow rate of each stream may range from about 0 to 50 kg/h.

Then, the first and second streams are pressurized at an elevated pressure in a combined stream with one or more intensifier pumps and delivered to the apparatus, causing the constituents of the first and second streams to interact within the apparatus at a nano/micro scale level. Preferably, the process pressure is, but not limited to, within the range of about 345 bar to about 3500 bar.

The selection of the mixing ratio, process pressure and solids concentration should be optimized to achieve the desired particle size. Preferably, the mixing ratio of the solvent to anti-solvent ratio is, but not limited to, in the range of about 1:2 to 1:50. Preferably, the solids concentration in the solvent mixture is, but not limited to, in the range of about 1 to 30 % w/w.

Co-precipitation conditions, such as the solvent/anti-solvent system and the mixing conditions, preferably determine the amorphous nature, the particle size and the morphology of the solids produced. The ratio of solvent to anti-solvent is dependent on the characteristics of the solvents and the substances used, such as supersaturation capacity of the solvents and precipitation rates of the substances. Preferably, the anti-solvent ratios vary between 2 and 30 times that of the solvent.

Preferably, the control of the temperature of the solvent and anti-solvent systems is used to manipulate both the supersaturation capacity and the precipitation rate of the substances. Preferably, the temperature of the solvent and/or anti-solvent system with the constituents is, but not limited to, within the range of -10°C to 50°C.

Stage (iii) - The method of the present invention comprises an optional isolation step (5) to separate the amorphous nanoparticles in the form of a powder (6), by removing the solvent from the resulting solid dispersion comprising the active drug and the stabilizing agent. The solvents may be removed by any suitable technology known to the skilled person in the art. Preferably, the step of removing the solvents comprises distillation, drying, spray drying, filtration, or any combination thereof. The morphology of the amorphous solid dispersed particles obtained can also be controlled during the isolation process parameters. For example, the morphology of the amorphous solid dispersed particles, such as the agglomeration level, the porosity of the aggregates and the bulk density of the powder may be controlled, preferably through the atomization used in the spray drying process and/or the drying process at a temperature to remove the excess residual solvent in the final product.

The method of the present invention includes preparing a solution comprising at least one pharmaceutically active compound and a solution comprising at least one stabilizing agent. Preferably, a solution comprising both the pharmaceutically active compound and the stabilizing agent is prepared.

The pharmaceutically active compound and the stabilizing agent are capable of forming amorphous co-precipitates in at least one solvent and/or anti-solvent system. The solution or solutions is then mixed with a second solvent by means of microfluidization or microreaction to obtain a suspension of amorphous particles by co-precipitation. The second solvent comprises at least one anti-solvent of the pharmaceutically active compound and/or the stabilizing agent. The amorphous particles may be isolated in the form of a powder in a known manner. The amorphous particles are nanoparticles with a particle size in the submicron range. The term "submicron range" in the context of the invention refers to a particle size in the range of a few nm to less than 1 mm.

The particle size of the amorphous nanoparticles may be in the range of from about 50 nm to about 10 µm; preferably in the range of from about 50 nm to about 1µm; and more preferably in the range of from about 50 nm to about 500 nm.

In one preferred aspect, the method comprises: (i) preparing a solution comprising at least one pharmaceutically active compound using a first solvent and a solution comprising at least one stabilizing agent using a second solvent; wherein the second solvent is an anti-solvent of the pharmaceutically active compound; and (ii) mixing the solutions by means of microfluidization or a microreaction to obtain a suspension of amorphous particles by co-precipitation.

In one preferred aspect, the method comprises: (i) preparing a solution comprising at least one pharmaceutically active compound and at least one stabilizing agent using a first solvent; and (ii) mixing the solution with a second solvent which comprises at least one anti-solvent of the pharmaceutically active compound and the stabilizing agent, by means of microfluidization or a microreaction to obtain a suspension of amorphous particles by co-precipitation.

In one preferred aspect, the method comprises: (i) preparing a solution comprising at least one pharmaceutically active compound and a solution comprising at least one stabilizing agent, wherein each of the solutions is prepared using a first solvent; and (ii) mixing the solutions with a second solvent which comprises at least one anti-solvent of the pharmaceutically active compound and the stabilizing agent by means of microfluidization or a microreaction to obtain a suspension of amorphous particles by co-precipitation. Preferably, the solutions are combined to form a single stream prior to mixing with the second solvent.

Preferably, the solution comprising at least one pharmaceutically active compound and the solution comprising at least one stabilizing agent are combined to form a first stream, prior to mixing with the second solvent by means of micro-fluidization or micro-reaction. Preferably, the second solvent is an anti-solvent of both the pharmaceutically active ingredient and the stabilizing agent.

Preferably, the solution comprising the stabilizing agent is combined with the second solvent to form a second stream. The second stream may comprise an anti-solvent of the pharmaceutically active compound. In this case, the solution comprising the pharmaceutically active compound forms a first stream.

The stabilizing agent used in the method of the present invention comprises at least one polymer and/or at least one surfactant. The polymer and/or surfactant may be present in an amount in the range of about 0.001 to 90 % (w/w) of the solid dispersion. The surfactant is preferably selected from the group comprising: an anionic surfactant, a cationic surfactant, a nonionic surfactant, and a combination thereof.

Preferably, the first stream comprising the pharmaceutical active compound and the stabilizing agent is combined with the second stream comprising the anti-solvent of the pharmaceutical active compound and the stabilizing agent, at a pressure sufficient to cause interaction of the constituents in the streams; and delivered to the one or more channels in the reaction chambers such that the constituents in the streams react at a micro and/or a nano scale to form a suspension of amorphous nanoparticles by co-precipitation.

Preferably, wherein the first stream comprising the pharmaceutical active compound, is combined with the second stream comprising the stabilizing agent and the anti-solvent of pharmaceutical active compound, at a pressure sufficient to cause interaction of the constituents in the streams and delivered to the one or more channels in the reaction chambers such that the constituents in the streams react at a micro and/or a nano scale to form a suspension of amorphous nanoparticles by co-precipitation.

The method of the present invention may further comprise the step of cooling or quenching the combined streams after interaction within the MRT or microreactor.

The present disclosure also relates to a particulate amorphous solid dispersion comprising 5 to 95 % (w/w) of the pharmaceutically active component and 95 to 5 % (w/w) of the stabilizing agent, which are preferably surfactants and/or polymers. The particulates may have a particle size in the range of about 50 nm to about 10 µm, preferably in the range of about 50 nm to about 10 µm, more preferably in the range of 50 nm to about 500 nm.

Preferably, the particulates of the solid dispersion have a bulk density in the range of from about 0.1 g/ml to 1.0 g/ml.

An organic compound for use in the process of this invention is any organic chemical entity whose solubility decreases from one solvent to another. This organic compound is preferably one or more pharmaceutically active compounds. Examples of preferred pharmaceutically active compounds may include, but not exclusively, poorly soluble active compounds, thermolabile compounds with poor stability, or drug products requiring small particle size and high densities.

In a preferred aspect, the definition of "low solubility", "poorly soluble" and "poorly water soluble" compounds corresponds to that of the Biopharmaceutics Classification System (BCS). According to the BCS, compounds can be divided in four classes, regarding solubility (according to the United States Pharmacopeia) and intestinal permeability. Class I compounds possess high permeability and high solubility, Class II compounds possess high permeability and low solubility, Class III compounds are characterized by low permeability and high solubility and Class IV compounds possess low permeability and low solubility. Poorly soluble compounds correspond to Class II and Class IV.

Examples of poorly soluble compounds include, but are not limited to: antifungal agents like intraconazole or a related drug, such as fluoconazole, terconazole, ketoconazole and saperconazole; anti-infective drugs, such as griseofulvin and related compounds (e.g. griseoverdin); anti malaria drugs (e.g. Atovaquone); protein kinase inhibitor like Afatinib, Axitinib,Bosutinib, Cetuximab,Crizotinib, Dasatinib, Erlotinib, Fostamatinib, Gefitinib, Ibrutinib, Imatinib, Zemurasenib, Lapatinib, Lenvatinib, Mubritinib or Nilotinib; immune system modulators (e.g. cyclosporine); cardiovascular drugs (e.g. digoxin and spironolactone); ibuprofen; sterols or steroids; drugs from the group comprising danazol, acyclovir, dapsone, indinavir, nifedipine, nitrofurantion, phentytoin, ritonavir, saquinavir, sulfamethoxazole, valproic acid, trimethoprin, acetazolamide, azathioprine, iopanoic acid, nalidixic acid, nevirapine, praziquantel, rifampicin, albendazole, amitrptyline, artemether, lumefantrine, chloropromazine, ciprofloxacin, clofazimine, efavirenz, iopinavir, folic acid, glibenclamide, haloperidol, ivermectin, mebendazole, niclosamide, pyrantel, pyrimethamine, retinol vitamin, sulfadiazine, sulfasalazine, triclabendazole, and cinnarizine.

A detailed listing of groups of preferred poorly soluble compounds includes, but is not limited to: active agents or bioactive compounds of the group of ACE inhibitors, adenohypophoseal hormones, adrenergic neuron blocking agents, adrenocortical steroids, inhibitors of the biosynthesis of adrenocortical steroids, alpha-adrenergic agonists, alpha-adrenergic antagonists, selective α₂-adrenergic agonists, analgesics, antipyretics and anti-inflammatory agents, androgens, anesthetics, antiaddictive agents, antiandrogens, antiarrhythmic agents, antiasthmatic agents, anticholinergic agents, anticholinesterase agents, anticoagulants, antidiabetic agents, antidiarrheal agents, antidiuretics, antiemetic and prokinetic agents, antiepileptic agents, antiestrogens, antifungal agents, antihypertensive agents, antimicrobial agents, antimigraine agents, antimuscarinic agents, antineoplastic agents, antiparasitic agents, antiparkinsons agents, antiplatelet agents, antiprogestins, antithyroid agents, antitussives, antiviral agents, antidepressants, azaspirodecanediones, barbituates, benzodiazepines, benzothiadiazides, beta-adrenergic agonists, beta-adrenergic antagonists, selective β₁-adrenergic antagonists, selective β₂-adrenergic agonists, bile salts, agents affecting volume and composition of body fluids, butyrophenones, agents affecting calcification, calcium channel blockers, cardiovascular drugs, catecholamines and sympathomimetic drugs, cholinergic agonists, cholinesterase reactivators, dermatological agents, diphenylbutylpiperidines, diuretics, ergot alkaloids, estrogens, ganglionic blocking agents, ganglionic stimulating agents, hydantoins, agents for control of gastric acidity and treatment of peptic ulcers, haematopoietic agents, histamines, histamine antagonists, 5-hydroxytryptamine antagonists, drugs for the treatment of hyperlipoproteinemia, hypnotics and sedatives, immunosuppressive agents, laxatives, methylxanthines, monoamine oxidase inhibitors, neuromuscular blocking agents, organic nitrates, opiod analgesics and antagonists, pancreatic enzymes, phenothiazines, progestins, prostaglandins, agents for the treatment of psychiatric disorders, retinoids, sodium channel blockers, agents for spasticity and acute muscle spasms, succinimides, thioxanthines, thrombolytic agents, thyroid agents, tricyclic antidepressants, inhibitors of tubular transport of organic compounds, drugs affecting uterine motility, vasodilators, vitamins and the like, alone or in combination.

Preferably, the pharmaceutically active compound is a tyrosine kinase inhibitor. For example, this may be selected from the group comprising: axitinib, crizotinib, dasatinib, erlotinib, gefitinib, imatinib, lapatinib, nilotinib, pazopanib, regorafenib, ruxolitinib, sorafenib, sunitinib, vandetanib, vemurafenib, and combinations thereof.

Preferred examples of the pharmaceutically active compound include, but limited to, itraconazole, cinnarizine, nilotinib, carbamazepine or a combination thereof.

Preferably, the pharmaceutically active compound is nilotinib.

The pharmaceutically active compound may be present in an amount in the range of about 0.1 to about 95% (w/w) of the dispersion.

The solvent used in the method, according to the present invention, is preferably a solvent or mixture of solvents in which one or more organic compounds, preferably pharmaceutically active compounds, of interest are at least partially soluble. Preferably, the solvent or mixture of solvents is also one in which excipients such as stabilizing agent (polymers/surfactants) are at least partially soluble. The solvent may be provided with one or more surface modifiers (surfactants), which are preferably an anionic surfactant, a cationic surfactant or a nonionic surfactant. Surfactants are compounds that lower the surface tension (or interfacial tension) between two liquids or between a liquid and a solid. Surfactants may also act as detergents, wetting agents, emulsifiers, foaming agents, and/or dispersants.

Examples of such solvents include, but are not limited to: water, acetone, methylchloride, dimethylformamide, methanol, ethanoldimethyl sulfoxide, methylethylketone, dimethylacetamide, lactic acid, isopropanol, 3-pentanol, n-propanol, glycerol, butylene glycol, ethylene glycol, propylene glycol, dimethyl isosorbide, tetrahydrofuran, 1,4-dioxanepolyethylene glycol, polyethylene glycol esters, polyethylene glycol sorbitans, polyethylene glycol monoalkyl ethers, polypropylene glycol, polypropylene alginate, butanediol or a mixture thereof.

The anti-solvent, according to the present invention, may be miscible or immiscible with the solvent and the substances present show low solubility or completely insoluble upon mixing. The preferred anti-solvent is, but not exclusively, an aqueous solution which may be provided with one or more surface modifiers such as an anionic surfactant, a cationic surfactant or a nonionic surfactant mixed in it. Preferably, the aqueous solution comprises deionized water.

Preferably, at least one surfactant is also used as a stabilization agent. The surfactant is an anionic surfactant, a cationic surfactant, a nonionic surfactant or a combination thereof.

Suitable anionic surfactants include, but are not limited to, potassium laurate, sodium lauryl sulfate, sodium dodecylsulfate, alkyl polyoxyethylene sulfates, sodium alginate, dioctyl sodium sulfosuccinate, phosphatidyl choline, phosphatidyl glycerol, phosphatidyl inosine, phosphatidylserine, phosphatidic acid and their salts, sodium carboxymethylcellulose, cholic acid and other bile acids (e.g., cholic acid, deoxycholic acid, glycocholic acid, taurocholic acid, glycodeoxycholic acid) and salts thereof (e.g., sodium deoxycholate, etc.) or a combination thereof.

Suitable cationic surfactants include, but are not limited to, quaternary ammonium compounds, such as benzalkonium chloride, cetyltrimethylammonium bromide, lauryldimethylbenzylammonium chloride, acyl carnitine hydrochiorides, or alkyl pyndinium halides.

Suitable nonionic surfactants include, but are not limited to, polyoxyethylene fatty alcohol ethers, polyoxyethylene sorbitan fatty acid esters, polyoxyethylene fatty acid esters, sorbitan esters, glycerol monostearate, polyethylene glycols, polypropylene glycols, cetyl alcohol, cetostearyl alcohol, stearyl alcohol, aryl alkyl polyether alcohols, polyoxyethylene-polyoxypropylene copolymers (poloxomers), polaxamines, methylcellulose, hydroxycellulose, hydroxy propylcellulose, hydroxy propylmethylcellulose, noncrystalline cellulose, polyvinyl alcohol, glyceryl esters, and polyvinylpyrrolidone or a combination thereof.

Preferably, a pH adjusting agent may be added to the anti-solvent solution. Examples of a pH adjusting agent includes, but is not limited to, sodium hydroxide, hydrochloric acid, tris buffer or citrate, acetate, lactate, meglumine, or the like.

At least one polymer is used for stabilization of the amorphous form. Polymers suitable for use in the formulations of the present invention include cellulose ester, cellulose ether, polyalkylene oxide, polyacrylate, polymethacrylate, polyacrylamide, polyvinyl alcohol, vinyl acetate polymer, oligosaccharide, polysaccharide, hydroxypropylcellulose, polyvinylpyrrolidone, hydroxyalkylcelluloses, hydroxyalkylalkylcellulose, hydroxypropylmethylcellulose, cellulose phthalate, cellulose succinate, cellulose acetate phthalate, hydroxypropylmethylcellulose phthalate, hydroxypropylmethylcellulose acetate succinate, polyethylene oxide, polypropylene oxide, copolymer of ethylene oxide and propylene oxide, methacrylic acid/ethyl acrylate copolymer, methacrylic acid/methyl methacrylate copolymer, hydroxypropylmethylcellulose succinate, butyl methacrylate/2-dimethylaminoethyl methacrylate copolymer, poly(hydroxyalkyl acrylate), poly(hydroxyalkyl methacrylate), Gelatin, , gelatin, copolymer of vinyl acetate and crotonic acid, partially hydrolyzed polyvinyl acetate, carrageenan, galactomannan, high viscosity gums or xanthan gum, or a combination thereof.

In addition to the active compounds and the stabilizing agents, the mixture may also include additional agents for improving the performance of the formulation. These may comprise, but not exclusively, compounds having a plasticizing effect or compounds having properties that improve the dissolution profile of the active substance.

Particles obtained by the method of the present invention may be formulated into a pharmaceutical composition. Examples of pharmaceutical forms for administration of amorphous solid dispersions synthesized through the method herein described may include solid dosage forms, such as tablets, capsules, granules, pellets or powders. The compositions obtained may have an enhanced performance including, but not exclusively, supersaturation, dissolution rate improvement, controlled release or taste masking.

The invention generally relates to a method to manufacture amorphous solid dispersions in a particulate form comprising: preparation of a solution comprising at least one active pharmaceutical ingredient and a stabilizing agent that are able to form amorphous co-precipitates in a solvent; mixing the solution with at least an anti-solvent by means of a microreactor obtaining by co-precipitation a suspension of amorphous nanoparticles; optionally, the method may comprise an isolation step to separate the particles in the form of a powder. The interaction of the solutions and the anti-solvent in the microreactor is effective to define the amorphous nature of the nano-suspension. The mixing within the microreactor is promoted by means of microfluidization and cavitation. Preferably, the solution and anti-solvents mixing is performed at high pressures.

Compared to the conventional methods for producing amorphous solid dispersion known in the prior art, the method of the present invention exhibits several advantages. The conventional methods for producing amorphous solid dispersions, e.g. spray drying and hot-melt extrusion, are not suitable for manufacturing compounds with low solubility in volatile organic solvents and/or with high melting points. The method of the present invention can use a wide range of solvent/anti-solvent systems while avoiding the use of high processing temperatures. Process and formulation conditions such as mixing energy, solvent/anti-solvent ratio, temperature, residence time, composition and concentration, can be manipulated to achieve the desired particle properties, such as powder density, particle size, surface area and dissolution rate. The suspended particles obtained by the method of the present invention are consistently in the amorphous solid state. Also, the particulate amorphous solid dispersion produced by the method of present invention is in submicron range, which is stable and has high density.

Furthermore, the amorphous solid dispersions obtained by the method of the present invention are in the stable particulate form, avoiding subsequent stabilization steps.

Particle size of the nanoparticles obtained by the method of the present invention is within the submicron range, avoiding subsequent high-energy processing that can lead to solid-state changes, e.g. milling, high shear mixing.

Also, in the present invention the isolation of the particles may be performed while maintaining powder characteristics. The isolation of the particles may also be performed to adjust/improve powder characteristics. One more advantage is that the process can be adapted to continuous processing and it is easily scalable.

The present invention also relates to a pharmaceutical composition comprising a particulate amorphous solid dispersion according to the present invention.

The present invention also relates to a pharmaceutical composition comprising a particulate amorphous solid dispersion according to the present invention for use as a medicament.

The present invention also relates a particulate amorphous solid dispersion for use in increasing the bioavailability of a pharmaceutically active compound.

Suitable examples, which are meant only to suggest a method of practicing the present invention and do not serve to limit the scope of the present invention, follows:

### EXAMPLE 1

Two separate solutions of Itraconazole (BCS/DCS Class IIb, Tm/Tg ~1.32, Log P 4.4) and 1:1 methacrylic acid - methyl methacrylate copolymer (Eudragit^{®} L100, Evonik Röhm GmbH, Darmstadt, Germany) were prepared. Both components, in a weight proportion 10:90 (total mass of 2 g) and 40:60 (total mass of 1 g), were dissolved in independent mixtures of ethanol/acetone in a volume proportion of 1:1. The total volume of solvent was 100 mL, thus solids concentration in one of the solutions was ~2.5 wt.%, while in the other was ~1.3 wt.%, respectively. As anti-solvent, a mass of deionized water corresponding to 10 times that of the solvent was used. The pH of the water was adjusted to 2.10 using a solution of hydrochloric acid (37%) and its temperature was reduced to 5 ± 2 °C.

Co-precipitates were produced using a PureNano^{™} Microfluidics Reaction Technology (Model MRT CR5) comprising a chamber with 75 µm diameter reaction channels followed by a chamber with 200 µm diameter reaction channels. The peristaltic pump was set to maintain a ratio of 1:10 of solvent and anti-solvent. The intensifying pump was set to impose a pressure of approximately 20 kPsi.

Following the co-precipitation process, the suspensions obtained were filtrated using vacuum and the wet cake was dried in a tray dryer oven at a temperature around 70°C during ~48h. At the end of the drying process, the powders were analyzed by X-ray powder diffraction (XRPD) for solid-state analysis, *i.e.* to evaluate the potential presence of crystalline material.

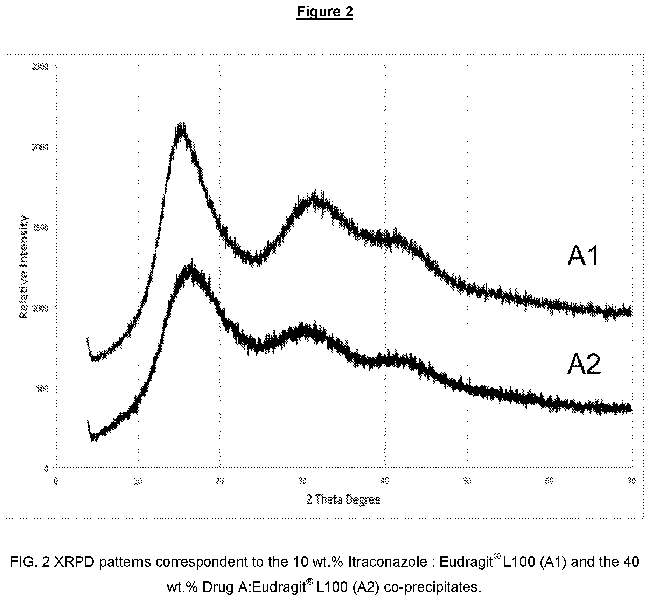

FIG.2 shows the XRPD patterns correspondent to the 10 wt.% and 40 wt.% drug load Itraconazole:Eudragit^{®} L100 co-precipitates (A1 and A2 spectra, respectively). XRPD experiments were performed in a D8 Advance Bruker AXS Theta-2Theta diffractometer with a copper radiation source (Cu Kalpha2, wavelength = 1.5406 Å), voltage 40 kV, and filament emission 35 mA. The samples were measured over a 2θ interval from 3 to 70° with a step size of 0.017° and step time of 50s.

Both formulations showed a halo characteristic of the amorphous form. No characteristic peaks of the XRPD profile of pure crystalline Itraconazole were observed in the freshly prepared products. These results indicated that amorphization of Itraconazole was successful when using the method described in the present invention. Moreover, and thanks to the presence of the polymer that also confers stabilization, amorphous formulations up to 40 wt.% drug load were able to be produced.

### EXAMPLE 2

A solution of Cinnarizine (BCS Class II, Tm/Tg ~1.40, Log P ~5.77) and 1:1 methacrylic acid - methyl methacrylate copolymer (Eudragit^{®} L100, Evonik Röhm GmbH, Darmstadt, Germany) was prepared. Both components, in a weight proportion 10:90 (total mass of 2 grams), were dissolved in a mixture of ethanol/acetone in a volume proportion of 1:1. The total volume of solvent was 100 mL, thus solids concentration in solution was ~2.5 wt.%. As anti-solvent, a mass of deionized water corresponding to 10 times that of the solvent was used. The pH of the water was adjusted to 2.10 using hydrochloric acid (37%) and its temperature was reduced to 5 ± 2 °C. Further processing of the solution was identical to the procedure applied in EXAMPLE 1, with the exception that following the co-precipitation process, the resultant suspension was divided in two equal parts - one part of the suspension was filtrated and dried in a tray dryer oven (same conditions as in EXAMPLE 1), while the second part was dried in a lab-scale spray dryer (*Büchi,* model B-290), equipped with a two fluid nozzle, to demonstrate that the isolation step does not affect the final drug's solid state and physical stability. The spray drying unit was operated in open cycle mode (*i.e.,* without recirculation of the drying gas). Before feeding the suspension to the nozzle, the spray drying unit was stabilized with nitrogen to assure stable inlet (T_in=141°C) and outlet temperatures (T_out=80°C). After stabilization, the suspension was fed to the nozzle by means of a peristaltic pump (F_feed=0.44 kg/h), and atomized at the nozzle's tip (F_atom=1.4 kg/h). The droplets were then dried in the spray drying chamber by a co-current nitrogen stream (F_drying=35 kg/h). The stream containing the dried particles was directed into a cyclone and collected at the bottom. At the end of the process, both products were analyzed by XRPD (same experimental method as in EXAMPLE 1), while only the spray-dried suspension was analyzed by scanning electron microscopy (SEM).

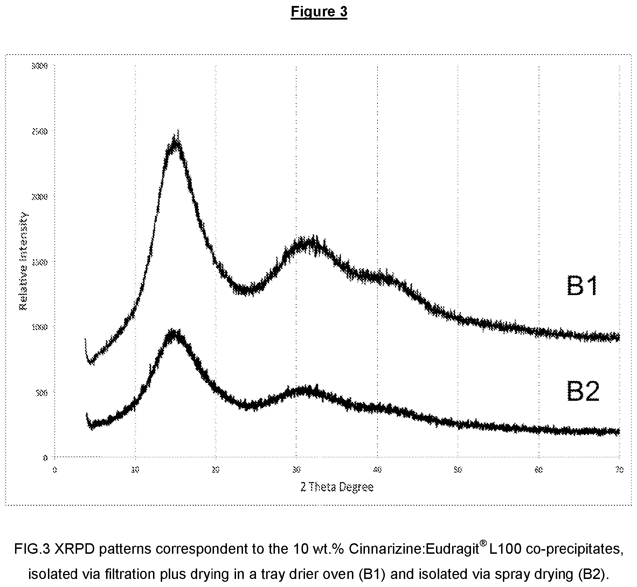

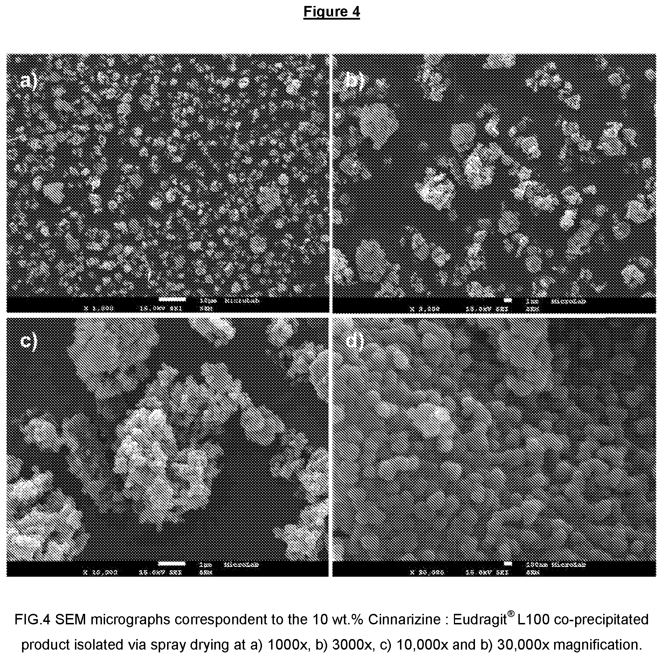

FIG.3 shows the XRPD patterns correspondent to the 10 wt.% Cinnarizine:Eudragit^{®} L100 co-precipitates, isolated via filtration plus drying in a tray drier oven (B1) and isolated via spray drying (B2). No significant differences between both XRPD patterns were observed. Similarly as in EXAMPLE 1, both products showed a halo characteristic of the amorphous form and no characteristic peaks of the XRPD profile of pure crystalline Cinnarizine were observed. These results indicated that (1) the method described in the present invention can be applied to produce amorphous dispersions/amorphous solutions of different therapeutic molecules with different physicochemical properties and (2) drug's solid state in the formulation in not dependent on the isolation process chosen. The spray drying of suspensions works as a simple method for separating particles, thus not influencing the drug's solid state and physical stability in the formulation. FIG.4a to 4d shows the SEM micrographs correspondent to the 10 wt.% Cinnarizine:Eudragit^{®} L100 co-precipitated product isolated via spray drying at 1000x, 3000x, 10,000x and 30,000x magnification, respectively. Observing the particles surface under high magnification (FIG. 4c and 4d) revealed that the agglomerates consisted of individual particles, most of them with a diameter around 100 nm.

### EXAMPLE 3

Two separate solutions of Nilotinib (BCS Class IV, Tm/Tg ~1,28, cLog P ~4.8) and 1:1 methacrylic acid - methyl methacrylate copolymer (Eudragit^{®} L100, Evonik Röhm GmbH, Darmstadt, Germany) were prepared. Both components, in a weight proportion 10:90 (total mass of 2 g) and 40:60 (total mass of 1 g), were dissolved in independent solutions of pure ethanol (the total volume of solvent was 100 mL, thus solids concentration in one of the solutions was ~2.5 wt.%, while in the other was ~1.3 wt.%, respectively). As anti-solvent, a mass of deionized water corresponding to 10 times that of the solvent was used. The pH of the water was adjusted to 2.10 using hydrochloric acid (37%) and its temperature was reduced to 5 ± 2 °C. Further processing of the solutions was identical to the procedure applied in EXAMPLE 1 and EXAMPLE 2, followed by vacuum filtration and simple drying steps. At the end of the process, the resultant product was also characterized by XRPD according to the experimental method described in previous examples.

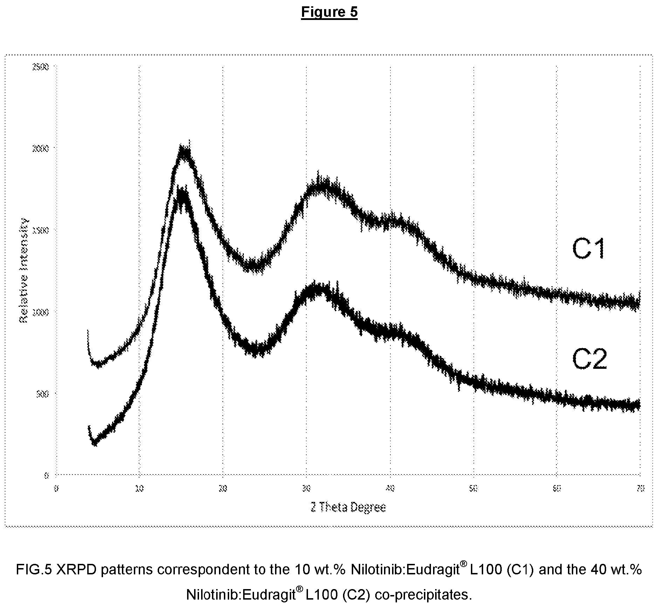

FIG.5 shows the XRPD patterns correspondent to the 10 wt.% and 40 wt.% drug load Nilotinib:Eudragit^{®} L100 co-precipitates (C1 and C2 spectra, respectively). Similarly as in EXAMPLE 1 and EXAMPLE 2, both formulations showed a halo characteristic of the amorphous form and no characteristic peaks of the XRPD profile of pure crystalline Nilotinib were observed in the freshly prepared products.

### EXAMPLE 4

An experiment with 40 wt.% Nilotinib and 60 wt.% Eudragit^{®} L100 was also produced following the conditions described above. Co-precipitates were isolated by spray drying using the process conditions described in EXAMPLE 2 - powders produced are hereafter named NanoAmorphous due to the particle in the submicron scale.

For comparison purposes, the same Nilotinib-based formulation was produced using a conventional approach by spray-drying - powders produced are hereafter named MicroAmorphous due to the particle in the micron scale. The experimental conditions were similar to the ones presented in EXAMPLE 2.

Both powders were compared in terms of their *in vitro* dissolution profile. Also, the dissolution profile of the crystalline state was evaluated. Powder dissolution profiles were obtained using a USP type II apparatus (DIS 6000, Copley Scientific, Nottingham, UK) in 900 mL of pH 6.5 FaSSIF biorelevent media (biorelevant, Croydon, UK) at a paddle rotation of 100 rpm, with a constant temperature bath at 37±0.5°C. The dissolution experiments were performed under non-sink conditions with a target drug load studied of 200 mg of Nilotinib. Sample aliquots (15 mL) were taken at various time points (15, 30, 60, 120 and 240 min) with no dissolution medium replacement. The aliquots were filtered immediately using a 0.45 µm filter (Acrodisc^{®} 25mm syringe filter with 0.45 µm hydrophilic polypropylene (GHP) membrane) and 4.5 mL of the filtrate was subsequently diluted with 0.5 mL of ethanol. In all cases, the filtrate was completely clear upon visual inspection before the quantification. The determination of the amount of Nilotinib in the media was performed using Beer's Law with a Hitachi's U-2000 Double-Beam UV/Vis spectrophotometer (Hitachi Ltd., Tokyo, Japan) at 265 nm.

Prior to the analysis of the *in vitro* dissolution results density measurements using a graduate cylinder were performed and it was observed that both bulk and tap density values of the NanoAmorphous powder were around two times the values obtained for the MicroAmorphous powder (*i.e.,* 0.432 g/mL versus 0.215 g/mL for the bulk density and 0.480 g/mL versus 0.239 g/mL for the tap density, respectively).

Spray drying in known by the production of hollow particles exhibiting low density and poor flowability. In opposition powder produced under the scope of this invention were solid/compact, with high bulk density and good flowability, ideal for the downstream processing i.e. tableting, capsule filling.

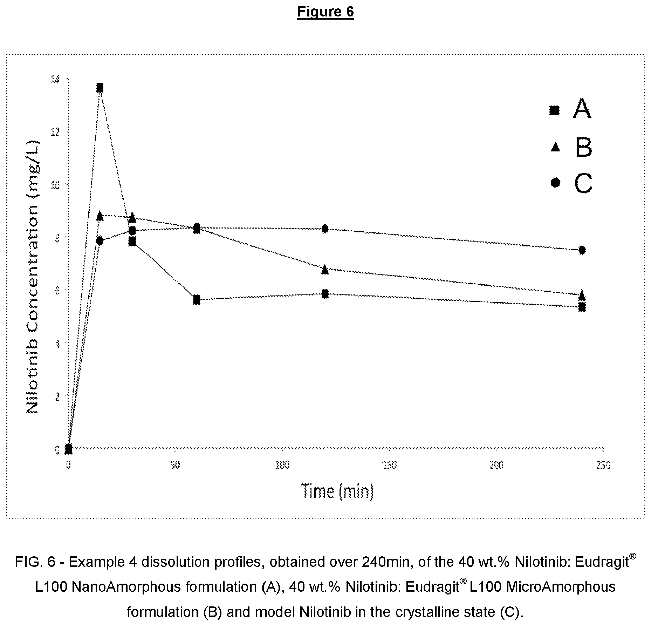

Figure 6 shows the powder dissolution profiles, obtained over 240min, of the 40 wt.% Nilotinib: Eudragit^{®} L100 NanoAmorphous formulation (A), 40 wt.% Nilotinib: Eudragit^{®} L100 MicroAmorphous formulation (B) and Nilotinib in the crystalline state (C).

As expected, the NanoAmorphous and MicroAmorphous formulations exhibited higher dissolution rates over the crystalline reference product.

When comparing NanoAmorphous vs MicroAmorphous, at the 15-minute time point, it was observed that the former formulation reached a significantly higher supersaturation level compared to the latter. The increase in the dissolution rate, due to the high-surface area of nanoparticles produced by the solvent controlled precipitation process, favored the creation of higher supersaturated levels when compared with amorphous micro-solid dispersions.

### EXAMPLE 5

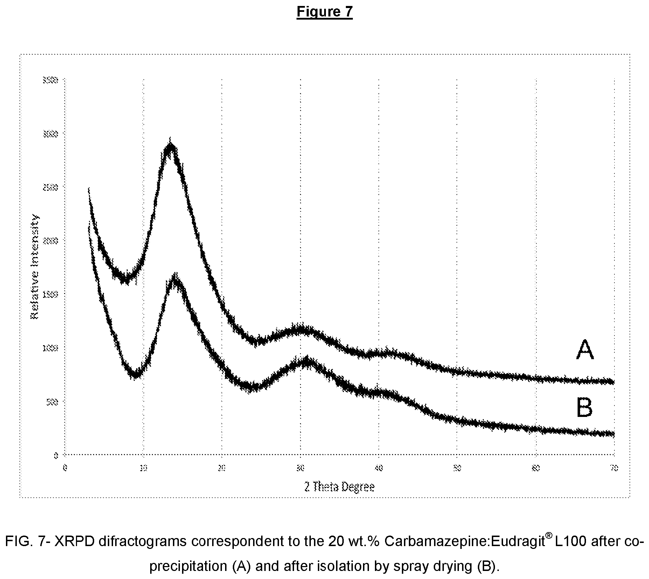

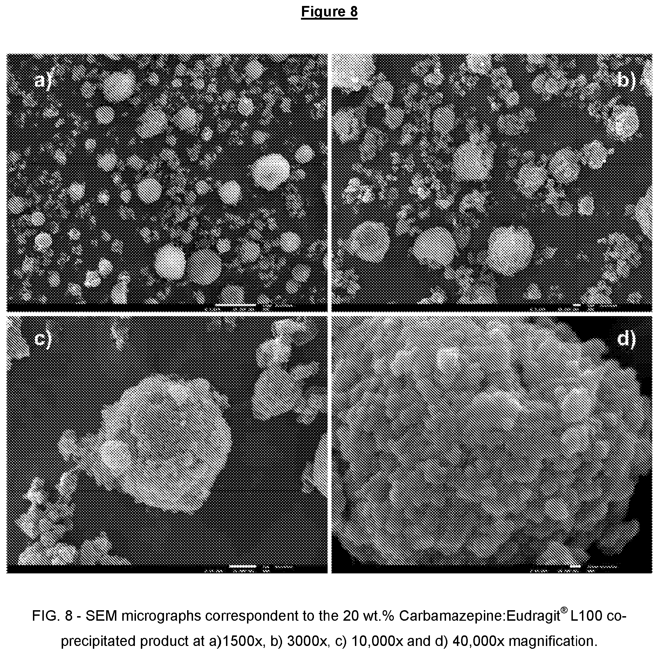

A solution of Carbamazepine (BCS/DCS Class Ila, Tm/Tg ~1,4, Log P ~2,6) and 1:1 methacrylic acid - methyl methacrylate copolymer (Eudragit^{®} L100, Evonik Röhm GmbH, Darmstadt, Germany) was prepared. Both components, in a weight proportion 20:80 (total mass of 3 grams), were dissolved in pure methanol (the total volume of solvent was 44 mL, thus solids concentration in solution was 8 wt.%). As anti-solvent, a mass of deionized water corresponding to 10 times that of the solvent was used. The pH of the water was adjusted to 2.10 using hydrochloric acid (37%) and its temperature was reduced to 5 ± 2 °C. Further processing of the solution was identical to the experimental procedure applied in previous examples, followed by a spray-drying step to isolate the particles. The spray drying unit was operated in open cycle mode (*i.e.,* without recirculation of the drying gas). Before feeding the suspension to the nozzle, the spray drying unit was stabilized with nitrogen to assure stable inlet (T_in ~156°C) and outlet temperatures (T_out ~80°C). After stabilization, the suspension was fed to the nozzle by means of a peristaltic pump (F_feed=0.81 kg/h), and atomized at the nozzle's tip (Atomization nitrogen, F_atom=1.4 kg/h). The droplets were then dried in the spray drying chamber by a co-current nitrogen stream (F_drying=40 kg/h). The stream containing the dried particles was directed into a cyclone and collected at the bottom. At the end of the process, the material produced was characterized by XRPD and SEM. The amorphous content is confirmed in Figure 7A. Similarly to previous examples the formulation exhibited a halo characteristic of the amorphous form and no signs of Carbamazepine crystallinity were observed. Figure 8 shows SEM micrographs with different magnifications. Agglomerated and spherical particles were obtained, similarly to the particles in EXAMPLE 2. However, in terms of particle size number distribution, it was observed a higher number of particles with a larger particle size in comparison with EXAMPLE 2 of about 100 nm.

To assess the *in vitro* performance of the amorphous nanocomposite particles produced by the method disclosed in the present invention, powder dissolution experiments were conducted.

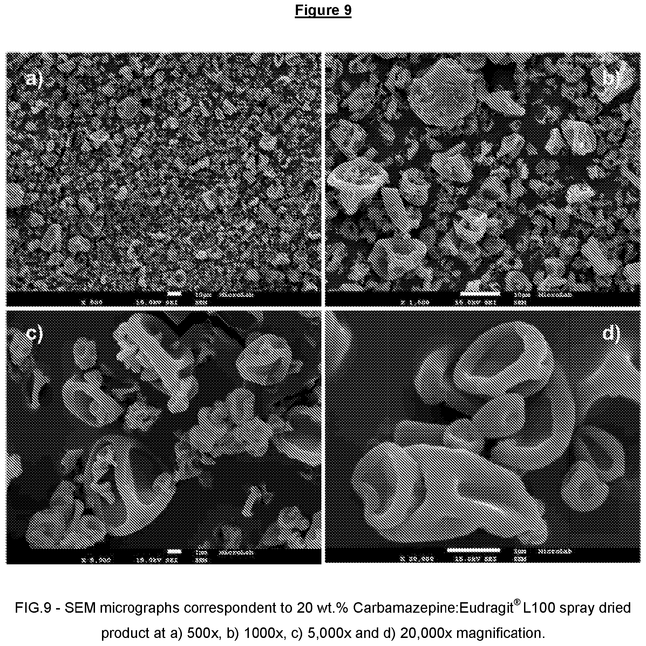

For comparison purposes, a formulation with 20 wt.% Carbamazepine:Eudragit^{®} L100 was produced following a conventional approach by spray drying. Process conditions were maintained constant. Similar XRPD difractograms were obtained as presented in Figure 7B. Similarly to Example 4, the typical particle size of amorphous solid dispersions produced by spray drying was limited to the micron size range (Figure 9).

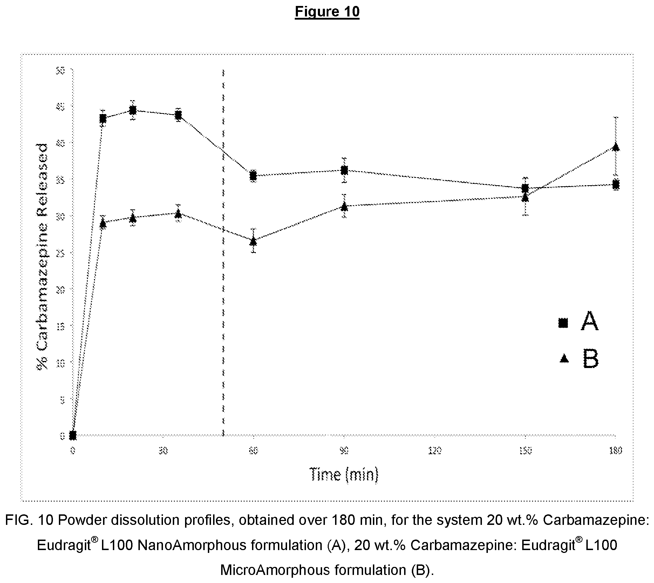

Figure 10 presents the powder in-vitro dissolution profiles for the materials produced under the scope of this invention (NanoAmorphous - A) and by spray drying (MicroAmorphous - B). The dashed line at the 50-minute time point corresponds to the pH-shift.

Powder dissolution profiles were obtained using a microcentrifuge pH-shift dissolution method. The experiments were conducted in 2 mL microcentrifuge tubes in a 37°C temperature water bath. The simulated gastric phase consisted of 0,9 mL of 0,01N HCl (pH=2) and the simulated intestinal phase consisted of an additional volume of 0,9 mL of FaSSIF biorelevent media (pH=6.5) (biorelevant, Croydon, UK). The combined pH value was verified using a pH strip (pH 6-6.5). Both media were degassed and preheated to 37 °C prior to use. The dissolution experiments were performed under non-sink conditions with a target drug load studied of 850 ug of Carbamazepine, which corresponded to approximately 5 and 2 times the equilibrium concentration of Carbamazepine in the gastric and intestinal phases, respectively. Sample aliquots were taken at various time points (10, 20, 35, 60, 90, 150 and 180 min) with no dissolution medium replacement. The pH-shift occurred at the 50-minute time point. The preparation of the test tubes for sampling consisted of removing the later from the water bath and centrifuged using an Himac Microcentrifuge CT15RE (Hitachi Koki Co, Ltd) for 1 minute at 13,000 rpm. Then, 25 uL of the supernatant was aliquoted, but only 10 uL diluted 15-fold in methanol in a HPLC vial with low volume insert (150 uL). The remaining solution was vortexed for a few seconds until total redispersion of the sediments was observed. The test tubes were placed back in the water bath until the next time point. The amount of Carbamazepine dissolved in the media was performed using a Waters (Model 2695) HPLC system with a photo-diode array detector (Model 2996). The column used was a Zorbax^{®} XDB - C18 (4.6 mm × 150 mm, 3.5 um) and the mobile phase was a 60:40 % v/v of methanol and water respectively. The injection volume was 10 uL and the flow rate was maintained constant at 1 mL/min. The UV absorbance was measured at 285 nm. The temperature of the column was maintained at 25∘C. The chromatographs were collected and integrated using Empower Version 2.0. The amount of drug in the samples was measured against a standard single-point injection.

Similarly to previous examples, due to the high-surface area of the amorphous nanoparticles produced under the scope of the present invention, dissolution rate was maximized compared with the MicroAmorphous produced by spray drying.

To understand the synergistic effect (nano + amorphous) in the absorption and bioavailability of DCS Class IIa drugs, pharmacokinetic studies with the NanoAmorphous and MicroAmorphous formulations were performed in mice.

Adult CD1 female mice (22-24 g) were purchased from Charles River (Barcelona, Spain). Animals were fed with standard laboratory food and water *ad libitum.* All animal experiments were performed with the approval of the local animal ethical committee, and in accordance with the Portuguese laws D.R. n° 31/92, D.R. 153 I-A 67/92, and all following legislations. On the day of dosing, the animals were fasted during approximately 6h before the start of the experiments. This period was considered sufficient for the emptying of the stomach in mice *(*Lab Anim. 2013, 47(4), 225-40*).* The mice were dosed by oral gavage with an equivalent amount of each formulation to provide 7.4 mg/kg body weight of Carbamazepine (n=3). The vehicle was acidified water (0,01N HCl, pH~2) and the concentration of the suspension was adjusted such that an appropriate dose was contained in 0.35 mL of the suspension. Being the stomach capacity of a mouse approximately 0.4 mL, 0.35 mL was considered an ideal oral dosage volume to not overload the stomach capacity and/or avoid reflux into the esophagus *(*J Pharm Pharmacol. 2008, 60(1), 63-70*).* The time interval between suspension preparation and dose administration was around 30 seconds. After dosing, the mice were kept in restraining cages, with free access to water. Blood samples (~ 1 mL aliquots) were collected from the orbital sinus at 2, 5, 10, 15, 30, 45, 60, 120 and 180 min post administration. The blood samples were centrifuged, and the serum samples were refrigerated until assayed. The concentration of Carbamazepine in the serum was assayed using an IMMULITE 2000^{®} XPi Immunoassay System (Siemens Healthcare Diagnostics). This system combines chemiluminescence and immunoassay reactions. The assay is based on the measurement of the light produced by dephosphorylation of a substrate, which is catalyzed by alkaline phosphatase (ALP), which in turn is directly conjugated to the drug in the sample. Thus, the light produced by the reaction is proportional to the amount of drug in the sample.

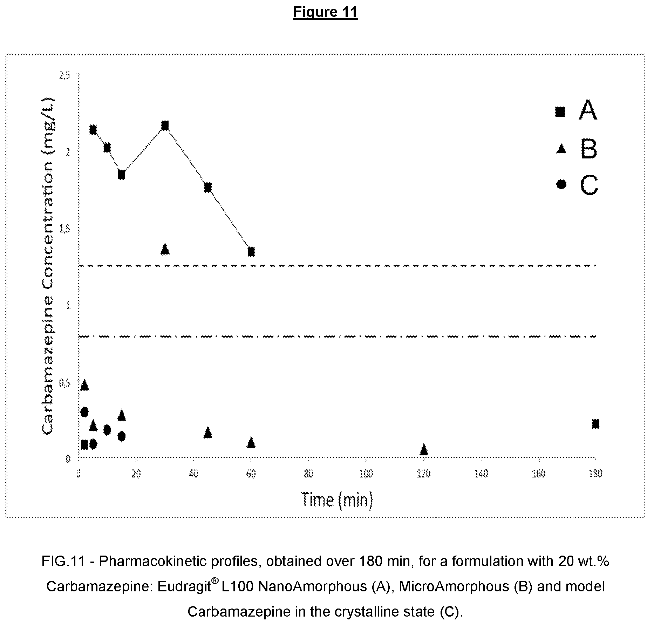

The pharmacokinetic profiles, obtained over 180 min, are presented in Figure 11 for the NanoAmorphous (A), MicroAmorphous (B) and Carbamazepine in the crystalline state (C). The dashed line corresponds to the limit of quantification (LOQ) of the immunoassay method, which is 1.25 mg/L. Thus, serum samples with an amount of Carbamazepine below the LOQ of the method were treated by a liquid-liquid extraction method and assayed using HPLC. Aliquots of serum were transferred to 2 mL microcentrifuge tubes. Methanol in a ratio 1:4 v/v was then added to each tube and vortex mixed for 5 min. White-opaque solutions were formed due to precipitation of water-soluble proteins. The samples were then centrifuged at 2,000 rpm for 5 min. The supernatants were extracted and directly transferred to HPLC vials with low volume inserts (150 uL). Each sample was analyzed using the previously described HPLC conditions. The dashed-dot line in Figure 11 corresponds to the maximum of Carbamazepine concentration obtainable in the serum samples, if a 60% yield is considered for the extraction process. This average yield value was determined by applying the extraction method to positives samples, *i.e.* samples that were above the LOQ of the immunoassay method.

Enhanced bioavailability in mice was observed with the NanoAmorphous system produced following the scope of the present invention when compared with the MicroAmorphous formulation or crystalline drug.

The observed differences can be explained with the difference in particle sizes among the formulations. The high surface area of the NanoAmorphous particles when exposed to gastrointestinal fluids led to very rapid dissolution rates, which in turn contributed to a greater amount of Carbamazepine in solution.

## Claims

1. A method of manufacturing amorphous solid dispersions in a particulate form, which method comprises:
(i) preparing a solution comprising at least one pharmaceutically active compound and a solution comprising at least one stabilizing agent, wherein each solution is prepared using a first solvent, and
(ii) mixing the solutions with a second solvent which comprises at least one anti-solvent
by means of microfluidization or a microreaction to obtain a suspension of amorphous particles by co-precipitation, wherein the stabilizing agent is at least one polymer selected from the group comprising: cellulose ester, cellulose ether, polyalkylene oxide, polyacrylate, polymethacrylate, polyacrylamide, polyvinyl alcohol, vinyl acetate polymer, oligosaccharide, polysaccharide, hydroxypropylcellulose, polyvinylpyrrolidone, hydroxyalkylcelluloses, hydroxyalkylalkylcellulose, hydroxypropylmethylcellulose, cellulose phthalate, cellulose succinate, cellulose acetate phthalate, hydroxypropylmethylcellulose phthalate, hydroxypropylmethylcellulose acetate succinate, polyethylene oxide, polypropylene oxide, copolymer of ethylene oxide and propylene oxide, methacrylic acid/ethyl acrylate copolymer, methacrylic acid/methyl methacrylate copolymer, hydroxypropylmethylcellulose succinate, butyl methacrylate/2-dimethylaminoethyl methacrylate copolymer, poly(hydroxyalkyl acrylate), poly(hydroxyalkyl methacrylate), gelatin, copolymer of vinyl acetate and crotonic acid, partially hydrolyzed polyvinyl acetate, carrageenan, galactomannan, high viscosity gums or xanthan gum, and a combination thereof, and/or at least one surfactant which comprises an anionic surfactant, a cationic surfactant, a nonionic surfactant or a combination thereof.

2. The method according to claim 1, wherein the solution comprising at least one pharmaceutically active compound and the solution comprising at least one stabilizing agent are combined to form a first stream, prior to mixing with the second solvent, optionally wherein the second solvent is an anti-solvent of both the pharmaceutically active ingredient and the stabilizing agent.

3. The method according to claim 1, wherein the solution comprising the stabilizing agent is combined with the second solvent to form a second stream, optionally wherein the second stream comprises an anti-solvent of the pharmaceutically active compound.

4. The method according to claim 4 or 5, wherein the solution comprising the pharmaceutically active compound forms a first stream.

5. The method according to claim 1, wherein the solution comprising at least one pharmaceutically active compound is prepared using a first solvent and the solution comprising at least one stabilizing agent is prepared using a second solvent; wherein the second solvent is an anti-solvent of the pharmaceutically active compound; and
the solutions are mixed by means of microfluidization or a microreaction to obtain a suspension of amorphous particles by co-precipitation.

6. The method according to any one of the preceding claims, further comprising an isolation step to separate the amorphous particles in the form of a powder, optionally wherein the amorphous particles are isolated by distillation, drying, spray drying, filtration or any combination thereof.

7. The method according to any one of the preceding claims, wherein the amorphous particles are nanoparticles having a particle size in submicron range, optionally wherein the particle size is in the range of about 50 nm to about 10 µm, or in the range of about 50 nm to about 1µm, or in the range of 50 nm to about 500 nm.

8. The method according to any one of the preceding claims, wherein the polymer and/or surfactant is present in an amount in the range of about 0.001 to 90 % (w/w) of the dispersion.

9. The method according to any one of claims 1 to 8, wherein the anionic surfactant is selected from the group comprising: potassium laurate, sodium lauryl sulfate, sodium dodecylsulfate, alkyl polyoxyethylene sulfates, sodium alginate, dioctyl sodium sulfosuccinate, phosphatidyl choline, phosphatidyl glycerol, phosphatidyl inosine, phosphatidylserine, phosphatidic acid and their salts, sodium carboxymethylcellulose, cholic acid, deoxycholic acid, glycocholic acid, taurocholic acid, glycodeoxycholic acid, and salts thereof, sodium deoxycholate, and a combination thereof; or
the cationic surfactant is selected from the group comprising: quaternary ammonium compounds (benzalkonium chloride), cetyltrimethylammonium bromide, lauryldimethylbenzylammonium chloride, acyl carnitine hydrochlorides, or alkyl pyridinium halides, and a combination thereof; or the nonionic surfactant is selected from the group comprising: polyoxyethylene fatty alcohol ethers, polyoxyethylene sorbitan fatty acid esters, polyoxyethylene fatty acid esters, sorbitan esters, glycerol monostearate, polyethylene glycols, polypropylene glycols, cetyl alcohol, cetostearyl alcohol, stearyl alcohol, aryl alkyl polyether alcohols, polyoxyethylene-polyoxypropylene copolymers (polaxamers), poloxamines, methylcellulose, hydroxycellulose, hydroxy propylcellulose, hydroxy propylmethylcellulose, noncrystalline cellulose, polyvinyl alcohol, glyceryl esters, and polyvinylpyrrolidone, and a combination thereof.

10. The method according to any one of the preceding claims, further comprising at least one of the following features:
(i) wherein the first solvent is the same or different for each solution;
(ii) wherein the first solvent and/or the second solvent comprises a mixture of solvents;
(iii) wherein the first and the second solvent is the same or different.

11. The method according to any one of the preceding claims, wherein the first and/or the second solvent is selected from the group comprising: water, acetone, methylchloride, dimethylformamide, methanol, ethanoldimethyl sulfoxide, methylethylketone, dimethylacetamide, lactic acid, isopropanol, 3-pentanol, n-propanol, glycerol, butylene glycol, ethylene glycol, propylene glycol, dimethyl isosorbide, tetrahydrofuran, 1,4-dioxanepolyethylene glycol, polyethylene glycol esters, polyethylene glycol sorbitans, polyethylene glycol monoalkyl ethers, polypropylene glycol, polypropylene alginate, butanediol, and mixtures thereof.

12. The method according to any one of the preceding claims, wherein the anti-solvent comprises an aqueous solution, optionally wherein the aqueous solution is a deionized water.

13. The method according to any one of the preceding claims, further comprising adding a pH adjusting agent to the anti-solvent, optionally wherein the pH adjusting agent is selected from the group comprising: sodium hydroxide, hydrochloric acid, tris buffer or citrate, acetate, lactate, meglumine, and a combination thereof.

14. The method according to any one of the preceding claims, wherein the pharmaceutically active compound is a tyrosine kinase inhibitor, optionally wherein the tyrosine kinase inhibitor is selected from the group comprising: axitinib, crizotinib, dasatinib, erlotinib, gefitinib, imatinib, lapatinib, nilotinib, pazopanib, regorafenib, ruxolitinib, sorafenib, sunitinib, vandetanib, vemurafenib, and combinations thereof.

15. The method according to any one of the preceding claims, wherein the pharmaceutically active compound is present in an amount in the range of about 0.1 to about 95% (w/w) of the dispersion.

16. The method according to any one of the preceding claims, wherein a plasticizing compound is added for improving the dissolution profile of the pharmaceutically active compound.

17. The method according to any one of the preceding claims, wherein the microfluidization or microreaction is effected using at least one microfluidics reaction technology (MRT) or a microreactor, optionally wherein the MRT and the microreactor comprises a reaction chamber.

18. The method according to claim 17, further comprising at least one of the following features:
(i) wherein the reaction chamber comprises one or more channels each having a diameter in the range of about 10 microns to about 400 microns, optionally the diameter of the channels is in range of about 50 microns to about 200 microns;
(ii) wherein the MRTs or microreactors are arranged in series or in parallel;
(iii) wherein the MRT or the microreactor is a continuous flow reactor.

19. The method according to claim 17 or 18, further comprising at least one of the following features:
(i) the solutions are continuously pumped into the reaction chamber where they are mixed and allowed to react (continuous flow reaction);
(ii) cooling or quenching the combined streams after interaction within the MRT and/or microreactor.

20. The method according to any one of claims 15 to 19, wherein the first stream comprising the pharmaceutically active compound and the stabilizing agent is combined with the second stream comprising the anti-solvent of the pharmaceutically active compound and the stabilizing agent, at a pressure sufficient to cause interaction of the constituents in the streams; and delivered to the one or more channels in the reaction chambers such that the constituents in the streams react to form a suspension of amorphous particles by co-precipitation, optionally wherein the pressure is in the range of about 345 bar to about 3500 bar.

21. The method according to any one of claims 15 to 19, wherein the first stream comprising the pharmaceutically active compound, is combined with the second stream comprising the stabilizing agent and the anti-solvent of pharmaceutically active compound, at a pressure sufficient to cause interaction of the constituents in the streams and delivered to the one or more channels in the reaction chambers such that the constituents in the streams react to form a suspension of amorphous particles by co-precipitation, optionally wherein the pressure is in the range of about 345 bar to about 3500 bar.

## Patentansprüche

1. Verfahren zur Fertigung von amorphen festen Dispersionen in einer partikulären Form, wobei das Verfahren Folgendes umfasst:
(i) Herstellen einer Lösung, umfassend mindestens eine pharmazeutisch aktive Verbindung, und einer Lösung, umfassend mindestens ein Stabilisierungsmittel, wobei jede Lösung unter Verwendung eines ersten Lösungsmittels hergestellt wird, und
(ii) Mischen der Lösungen mit einem zweiten Lösungsmittel, das mindestens ein Antilösungsmittel umfasst, mittels Mikrofluidisierung oder einer Mikroreaktion, um eine Suspension amorpher Partikel durch Kopräzipitation zu erlangen, wobei das Stabilisierungsmittel mindestens ein Polymer ist, das ausgewählt ist aus der Gruppe, umfassend: Celluloseester, Celluloseether, Polyalkylenoxid, Polyacrylat, Polymethacrylat, Polyacrylamid, Polyvinylalkohol, Vinylacetatpolymer, Oligosaccharid, Polysaccharid, Hydroxypropylcellulose, Polyvinylpyrrolidon, Hydroxyalkylcellulosen, Hydroxyalkylalkylcellulose, Hydroxypropylmethylcellulose, Cellulosephthalat, Cellulosesuccinat, Celluloseacetatphthalat, Hydroxypropylmethylcellulosephthalat, Hydroxypropylmethylcelluloseacetatsuccinat, Polyethylenoxid, Polypropylenoxid, Copolymer aus Ethylenoxid und Propylenoxid, Methacrylsäure-/Ethylacrylat-Copolymer, Methacrylsäure-/Methylmethacrylat-Copolymer, Hydroxypropylmethylcellulosesuccinat, Butylmethacrylat-/2-Dimethylaminoethylmethacrylat-Copolymer, Poly(hydroxyalkylacrylat), Poly(hydroxyalkylmethacrylat), Gelatine, Copolymer aus Vinylacetat und Crotonsäure, teilweise hydrolysiertes Polyvinylacetat, Carrageen, Galactomannan, hochviskose Gummis oder Xanthangummi und eine Kombination davon und/oder mindestens ein Tensid, das ein anionisches Tensid, ein kationisches Tensid, ein nichtionisches Tensid oder eine Kombination davon umfasst.

2. Verfahren nach Anspruch 1, wobei die Lösung, umfassend mindestens eine pharmazeutisch aktive Verbindung, und die Lösung, umfassend mindestens ein Stabilisierungsmittel, vor dem Mischen mit dem zweiten Lösungsmittel kombiniert werden, um einen ersten Strom zu bilden, wobei optional das zweite Lösungsmittel ein Antilösungsmittel sowohl des pharmazeutisch aktiven Inhaltsstoffs als auch des Stabilisierungsmittels ist.

3. Verfahren nach Anspruch 1, wobei die Lösung, umfassend das Stabilisierungsmittel, mit dem zweiten Lösungsmittel kombiniert wird, um einen zweiten Strom zu bilden, wobei optional der zweite Strom ein Antilösungsmittel der pharmazeutisch aktiven Verbindung umfasst.

4. Verfahren nach Anspruch 3, wobei die Lösung, umfassend die pharmazeutisch aktive Verbindung, einen ersten Strom bildet.

5. Verfahren nach Anspruch 1, wobei die Lösung, umfassend mindestens eine pharmazeutisch aktive Verbindung, unter Verwendung eines ersten Lösungsmittels hergestellt wird und die Lösung, umfassend mindestens ein Stabilisierungsmittel, unter Verwendung eines zweiten Lösungsmittels hergestellt wird; wobei das zweite Lösungsmittel ein Antilösungsmittel der pharmazeutisch aktiven Verbindung ist; und
die Lösungen mittels Mikrofluidisierung oder einer Mikroreaktion gemischt werden, um eine Suspension amorpher Partikel durch Kopräzipitation zu erlangen.

6. Verfahren nach einem der vorhergehenden Ansprüche, ferner umfassend einen Isolationsschritt, um die amorphen Partikel in der Form eines Pulvers zu trennen, wobei optional die amorphen Partikel durch Destillation, Trocknen, Sprühtrocknen, Filtration oder eine beliebige Kombination davon isoliert werden.

7. Verfahren nach einem der vorhergehenden Ansprüche, wobei die amorphen Partikel Nanopartikel sind, die eine Partikelgröße im Submikronbereich aufweisen, wobei optional die Partikelgröße im Bereich von etwa 50 nm bis etwa 10 µm oder im Bereich von etwa 50 nm bis etwa 1 µm oder im Bereich von 50 nm bis etwa 500 nm liegt.

8. Verfahren nach einem der vorhergehenden Ansprüche, wobei das Polymer und/oder Tensid in einer Menge im Bereich von etwa 0,001 bis 90 % (w/w) der Dispersion vorhanden ist.

9. Verfahren nach einem der Ansprüche 1 bis 8, wobei das anionische Tensid ausgewählt ist aus der Gruppe, umfassend: Kaliumlaurat, Natriumlaurylsulfat, Natriumdodecylsulfat, Alkylpolyoxyethylensulfate, Natriumalginat, Dioctylnatriumsulfosuccinat, Phosphatidylcholin, Phosphatidylglycerin, Phosphatidylinosin, Phosphatidylserin, Phosphatidsäure und ihre Salze, Natriumcarboxymethylcellulose, Cholsäure, Desoxycholsäure, Glycocholsäure, Taurocholsäure, Glycodesoxycholsäure und Salze davon, Natriumdesoxycholat und eine Kombination davon; oder das kationische Tensid ausgewählt ist aus der Gruppe, umfassend: quartäre Ammoniumverbindungen (Benzalkoniumchlorid), Cetyltrimethylammoniumbromid, Lauryldimethylbenzylammoniumchlorid, Acylcarnitinhydrochloride oder Alkylpyridiniumhalogenide und eine Kombination davon; oder
das nichtionische Tensid ausgewählt ist aus der Gruppe, umfassend: Polyoxyethylenfettalkoholether, Polyoxyethylensorbitanfettsäureester, Polyoxyethylenfettsäureester, Sorbitanester, Glycerinmonostearat, Polyethylenglycole, Polypropylenglycole, Cetylalkohol, Cetylstearylalkohol, Stearylalkohol, Arylalkylpolyetheralkohole, Polyoxyethylen-Polyoxypropylen-Copolymere (Poloxamere), Poloxamine, Methylcellulose, Hydroxycellulose, Hydroxypropylcellulose, Hydroxypropylmethylcellulose, nichtkristalline Cellulose, Polyvinylalkohol, Glycerylester und Polyvinylpyrrolidon und eine Kombination davon.

10. Verfahren nach einem der vorhergehenden Ansprüche, ferner umfassend mindestens eines der folgenden Merkmale:
(i) wobei das erste Lösungsmittel für jede Lösung gleich oder unterschiedlich ist;
(ii) wobei das erste Lösungsmittel und/oder das zweite Lösungsmittel ein Gemisch aus Lösungsmitteln umfasst;
(iii) wobei das erste und das zweite Lösungsmittel gleich oder unterschiedlich sind.

11. Verfahren nach einem der vorhergehenden Ansprüche, wobei das erste und/oder das zweite Lösungsmittel ausgewählt sind aus der Gruppe, umfassend: Wasser, Aceton, Methylchlorid, Dimethylformamid, Methanol, Ethanoldimethylsulfoxid, Methylethylketon, Dimethylacetamid, Milchsäure, Isopropanol, 3-Pentanol, n-Propanol, Glycerin, Butylenglycol, Ethylenglycol, Propylenglycol, Dimethylisosorbid, Tetrahydrofuran, 1,4-Dioxanpolyethylenglycol, Polyethylenglycolester, Polyethylenglycolsorbitane, Polyethylenglycolmonoalkylether, Polypropylenglycol, Polypropylenalginat, Butandiol und Gemische davon.

12. Verfahren nach einem der vorhergehenden Ansprüche, wobei das Antilösungsmittel eine wässrige Lösung umfasst, wobei optional die wässrige Lösung ein deionisiertes Wasser ist.

13. Verfahren nach einem der vorhergehenden Ansprüche, ferner umfassend Hinzufügen eines pH-Einstellmittels zu dem Antilösungsmittel, wobei optional das pH-Einstellmittel ausgewählt ist aus der Gruppe, umfassend: Natriumhydroxid, Salzsäure, Tris-Puffer oder -Citrat, Acetat, Lactat, Meglumin und eine Kombination davon.

14. Verfahren nach einem der vorhergehenden Ansprüche, wobei die pharmazeutisch aktive Verbindung ein Tyrosinkinaseinhibitor ist, wobei optional der Tyrosinkinaseinhibitor ausgewählt ist aus der Gruppe, umfassend: Axitinib, Crizotinib, Dasatinib, Erlotinib, Gefitinib, Imatinib, Lapatinib, Nilotinib, Pazopanib, Regorafenib, Ruxolitinib, Sorafenib, Sunitinib, Vandetanib, Vemurafenib und Kombinationen davon.

15. Verfahren nach einem der vorhergehenden Ansprüche, wobei die pharmazeutisch aktive Verbindung in einer Menge im Bereich von etwa 0,1 bis etwa 95 % (w/w) der Dispersion vorhanden ist.

16. Verfahren nach einem der vorhergehenden Ansprüche, wobei eine weichmachende Verbindung hinzugefügt wird, um das Auflösungsprofil der pharmazeutisch aktiven Verbindung zu verbessern.

17. Verfahren nach einem der vorhergehenden Ansprüche, wobei die Mikrofluidisierung oder Mikroreaktion unter Verwendung mindestens einer Mikrofluidik-Reaktionstechnologie (MRT) oder eines Mikroreaktors bewirkt wird, wobei optional die MRT und der Mikroreaktor eine Reaktionskammer umfassen.

18. Verfahren nach Anspruch 17, ferner umfassend mindestens eines der folgenden Merkmale:
(i) wobei die Reaktionskammer einen oder mehrere Kanäle umfasst, die jeweils einen Durchmesser im Bereich von etwa 10 Mikron bis etwa 400 Mikron aufweisen, optional der Durchmesser der Kanäle im Bereich von etwa 50 Mikron bis etwa 200 Mikron liegt;
(ii) wobei die MRTs oder Mikroreaktoren in Reihe oder parallel angeordnet sind;
(iii) wobei die MRT oder der Mikroreaktor ein Durchflussreaktor ist.

19. Verfahren nach Anspruch 17 oder 18, ferner umfassend mindestens eines der folgenden Merkmale:
(i) die Lösungen werden kontinuierlich in die Reaktionskammer gepumpt, wo sie gemischt und reagieren gelassen werden (Durchflussreaktion);
(ii) Abkühlen oder Quenchen der kombinierten Ströme nach Wechselwirkung innerhalb der MRT und/oder des Mikroreaktors.

20. Verfahren nach einem der Ansprüche 15 bis 19, wobei der erste Strom, umfassend die pharmazeutisch aktive Verbindung und das Stabilisierungsmittel, mit dem zweiten Strom, umfassend das Antilösungsmittel der pharmazeutisch aktiven Verbindung und das Stabilisierungsmittel, bei einem Druck kombiniert wird, der ausreicht, um eine Wechselwirkung der Bestandteile in den Strömen zu bewirken; und dem einen oder den mehreren Kanälen in den Reaktionskammern zugeführt wird, sodass die Bestandteile in den Strömen reagieren, um eine Suspension amorpher Partikel durch Kopräzipitation zu bilden, wobei optional der Druck im Bereich von etwa 345 bar bis etwa 3500 bar liegt.

21. Verfahren nach einem der Ansprüche 15 bis 19, wobei der erste Strom, umfassend die pharmazeutisch aktive Verbindung, mit dem zweiten Strom, umfassend das Stabilisierungsmittel und das Antilösungsmittel der pharmazeutisch aktiven Verbindung, bei einem Druck kombiniert wird, der ausreicht, um eine Wechselwirkung der Bestandteile in den Strömen zu bewirken, und dem einen oder den mehreren Kanälen in den Reaktionskammern zugeführt wird, sodass die Bestandteile in den Strömen reagieren, um eine Suspension amorpher Partikel durch Kopräzipitation zu bilden, wobei optional der Druck im Bereich von etwa 345 bar bis etwa 3500 bar liegt.

## Revendications

1. Procédé de fabrication de dispersions solides amorphes sous une forme particulaire, lequel procédé comprend :
(i) la préparation d'une solution comprenant au moins un composé pharmaceutiquement actif et d'une solution comprenant au moins un agent stabilisant, dans lequel chaque solution est préparée à l'aide d'un premier solvant, et
(ii) le mélange des solutions avec un second solvant qui comprend au moins un anti-solvant au moyen d'une microfluidisation ou d'une microréaction pour obtenir une suspension de particules amorphes par coprécipitation, dans lequel l'agent stabilisant est au moins un polymère choisi dans le groupe comprenant : un ester de cellulose, un éther de cellulose, un oxyde de polyalkylène, un polyacrylate, un polyméthacrylate, un polyacrylamide, un alcool polyvinylique, un polymère d'acétate de vinyle, un oligosaccharide, un polysaccharide, une hydroxypropylcellulose, une polyvinylpyrrolidone, des hydroxyalkylcelluloses, une hydroxyalkylalkylcellulose, une hydroxypropylméthylcellulose, un phtalate de cellulose, un succinate de cellulose, un phtalate d'acétate de cellulose, un phtalate d'hydroxypropylméthylcellulose, un succinate d'acétate d'hydroxypropylméthylcellulose, un oxyde de polyéthylène, un oxyde de polypropylène, un copolymère d'oxyde d'éthylène et d'oxyde de propylène, un copolymère d'acide méthacrylique/acrylate d'éthyle, un copolymère d'acide méthacrylique/méthacrylate de méthyle, un succinate d'hydroxypropylméthylcellulose, un copolymère de méthacrylate de butyle et de 2-diméthylaminoéthylméthacrylate, un poly(acrylate d'hydroxyalkyle), un poly (méthacrylate d'hydroxyalkyle), de la gélatine, un copolymère d'acétate de vinyle et d'acide crotonique, un acétate de polyvinyle partiellement hydrolysé, des gommes à haute viscosité carraghénanes, galactomannanes ou une gomme xanthane, et leurs combinaisons, et/ou au moins un tensioactif qui comprend un tensioactif anionique, un tensioactif cationique, un tensioactif non ionique ou une combinaison de ceux-ci.

2. Procédé selon la revendication 1, dans lequel la solution comprenant au moins un composé pharmaceutiquement actif et la solution comprenant au moins un agent stabilisant sont combinées pour former un premier flux, avant un mélange avec le second solvant, éventuellement dans lequel le second solvant est un anti-solvant à la fois de l'ingrédient pharmaceutiquement actif et de l'agent stabilisant.

3. Procédé selon la revendication 1, dans lequel la solution comprenant l'agent stabilisant est combinée avec le second solvant pour former un second flux, éventuellement dans lequel le second flux comprend un anti-solvant du composé pharmaceutiquement actif.

4. Procédé selon la revendication 3, dans lequel la solution comprenant le composé pharmaceutiquement actif forme un premier flux.

5. Procédé selon la revendication 1, dans lequel la solution comprenant au moins un composé pharmaceutiquement actif est préparée à l'aide d'un premier solvant et la solution comprenant au moins un agent stabilisant est préparée à l'aide d'un second solvant ; dans lequel le second solvant est un anti-solvant du composé pharmaceutiquement actif ; et
les solutions sont mélangées au moyen d'une microfluidisation ou d'une microréaction pour obtenir une suspension de particules amorphes par coprécipitation.

6. Procédé selon l'une quelconque des revendications précédentes, comprenant en outre une étape d'isolation pour séparer les particules amorphes sous la forme d'une poudre, éventuellement dans lequel les particules amorphes sont isolées par distillation, séchage, séchage par pulvérisation, filtration ou toute combinaison de ceux-ci.

7. Procédé selon l'une quelconque des revendications précédentes, dans lequel les particules amorphes sont des nanoparticules présentant une taille de particule dans le domaine submicronique, éventuellement dans lequel la taille de particule est dans la plage d'environ 50 nm à environ 10 µm, ou dans la plage d'environ 50 nm à environ 1 µm, ou dans la plage de 50 nm à environ 500 nm.

8. Procédé selon l'une quelconque des revendications précédentes, dans lequel le polymère et/ou l'agent tensioactif est présent en une quantité dans la plage d'environ 0,001 à 90 % (en poids) de la dispersion.

9. Procédé selon l'une quelconque des revendications 1 à 8, dans lequel le tensioactif anionique est choisi dans le groupe comprenant : le laurate de potassium, le laurylsulfate de sodium, le dodécylsulfate de sodium, les sulfates d'alkyl polyoxyéthylène, l'alginate de sodium, le sulfosuccinate de dioctyl sodium, la phosphatidyl choline, le phosphatidyl glycérol, la phosphatidyl inosine, la phosphatidylsérine, l'acide phosphatidique et des sels de celui-ci, la carboxyméthylcellulose de sodium, l'acide cholique, l'acide désoxycholique, l'acide glycocholique, l'acide taurocholique, l'acide glycodésoxycholique et des sels de celui-ci, le désoxycholate de sodium et une combinaison de ceux-ci ; ou
le tensioactif cationique est choisi dans le groupe comprenant : les composés d'ammonium quaternaire (chlorure de benzalkonium), le bromure de cétyltriméthylammonium, le chlorure d'iauryldiméthylbenzylammonium, les hydrochlorures d'acyl carnitine ou les halogénures d'alkyl pyridinium, et une combinaison de ceux-ci ; ou
le tensioactif non ionique est choisi dans le groupe comprenant : les éthers d'alcools gras de polyoxyéthylène, les esters d'acides gras de polyoxyéthylène sorbitane, les esters d'acides gras de polyoxyéthylène, les esters de sorbitane, le monostéarate de glycérol, les polyéthylène glycols, les polypropylène glycols, l'alcool cétylique, l'alcool cétostéarylique, l'alcool stéarylique, les alcools d'aryl alkyl polyéther, les copolymères de polyoxyéthylène-polyoxypropylène (poloxamères), les poloxamines, la méthylcellulose, l'hydroxycellulose, l'hydroxypropylcellulose, l'hydroxypropylméthylcellulose, la cellulose non cristalline, l'alcool polyvinylique, les esters de glycéryle et la polyvinylpyrrolidone, et une combinaison de ceux-ci.

10. Procédé selon l'une quelconque des revendications précédentes, comprenant en outre au moins l'une des caractéristiques suivantes :
(i) dans lequel le premier solvant est identique ou différent pour chaque solution ;
(ii) dans lequel le premier solvant et/ou le second solvant comprend un mélange de solvants ;
(iii) dans lequel le premier et le second solvant sont identiques ou différents.

11. Procédé selon l'une quelconque des revendications précédentes, dans lequel le premier et/ou le second solvant est choisi dans le groupe comprenant : l'eau, l'acétone, le chlorure de méthyle, le diméthylformamide, le méthanol, l'éthanoldiméthyl sulfoxyde, la méthyléthylcétone, le diméthylacétamide, l'acide lactique, l'isopropanol, le 3-pentanol, le n-propanol, le glycérol, le butylène glycol, l'éthylène glycol, le propylène glycol, le diméthyl isosorbide, le tétrahydrofurane, le 1,4-dioxanepolyéthylène glycol, les esters de polyéthylène glycol, les sorbitanes de polyéthylène glycol, les éthers monoalkyliques de polyéthylène glycol, le polypropylène glycol, l'alginate de polypropylène, le butanediol et des mélanges de ceux-ci.

12. Procédé selon l'une quelconque des revendications précédentes, dans lequel l'anti-solvant comprend une solution aqueuse, éventuellement dans lequel la solution aqueuse est une eau désionisée.

13. Procédé selon l'une quelconque des revendications précédentes, comprenant en outre l'ajout d'un agent d'ajustement du pH à l'anti-solvant, éventuellement dans lequel l'agent d'ajustement du pH est choisi dans le groupe comprenant : l'hydroxyde de sodium, l'acide chlorhydrique, le tampon tris ou le citrate, l'acétate, le lactate, la méglumine, et une combinaison de ceux-ci.

14. Procédé selon l'une quelconque des revendications précédentes, dans lequel le composé pharmaceutiquement actif est un inhibiteur de tyrosine kinase, éventuellement dans lequel l'inhibiteur de tyrosine kinase est choisi dans le groupe comprenant : axitinib, crizotinib, dasatinib, erlotinib, gefitinib, imatinib, Iapatinib, nilotinib, pazopanib, regorafenib, ruxolitinib, sorafenib, sunitinib, vandetanib, vemurafenib, et des combinaisons de ceux-ci.

15. Procédé selon l'une quelconque des revendications précédentes, dans lequel le composé pharmaceutiquement actif est présent en une quantité dans la plage d'environ 0,1 à environ 95 % (en poids) de la dispersion.

16. Procédé selon l'une quelconque des revendications précédentes, dans lequel un composé plastifiant est ajouté pour améliorer le profil de dissolution du composé pharmaceutiquement actif.

17. Procédé selon l'une quelconque des revendications précédentes, dans lequel la microfluidisation ou la microréaction est effectuée à l'aide d'au moins une technologie de réaction microfluidique (MRT) ou d'un microréacteur, éventuellement dans lequel la MRT et le microréacteur comprennent une chambre de réaction.

18. Procédé selon la revendication 17, comprenant en outre au moins l'une des caractéristiques suivantes :
(i) dans lequel la chambre de réaction comprend un ou plusieurs canaux présentant chacun un diamètre dans la plage d'environ 10 microns à environ 400 microns, éventuellement le diamètre des canaux est dans la plage d'environ 50 microns à environ 200 microns ;
(ii) dans lequel les MRT ou les microréacteurs sont agencés en série ou en dérivation ;
(iii) dans lequel la MRT ou le microréacteur est un réacteur à écoulement continu.

19. Procédé selon la revendication 17 ou 18, comprenant en outre au moins l'une des caractéristiques suivantes :
(i) les solutions sont pompées en continu dans la chambre de réaction où elles sont mélangées et autorisées à réagir (réaction en écoulement continu) ;
(ii) refroidissement ou trempage des flux combinés après interaction au sein de la MRT et/ou du microréacteur.

20. Procédé selon l'une quelconque des revendications 15 à 19, dans lequel le premier flux comprenant le composé pharmaceutiquement actif et l'agent stabilisant est combiné avec le second flux comprenant l'anti-solvant du composé pharmaceutiquement actif et l'agent stabilisant, à une pression suffisante pour entraîner une interaction des constituants dans les flux ; et délivré dans les un ou plusieurs canaux dans les chambres de réaction de sorte que les constituants dans les flux réagissent pour former une suspension de particules amorphes par coprécipitation, éventuellement dans lequel la pression est dans la plage d'environ 345 bars à environ 3 500 bars.

21. Procédé selon l'une quelconque des revendications 15 à 19, dans lequel le premier flux comprenant le composé pharmaceutiquement actif est combiné avec le second flux comprenant l'agent stabilisant et l'anti-solvant de composé pharmaceutiquement actif, à une pression suffisante pour entraîner une interaction des constituants dans les flux et délivré dans les un ou plusieurs canaux dans les chambres de réaction de sorte que les constituants dans les flux réagissent pour former une suspension de particules amorphes par coprécipitation, éventuellement dans lequel la pression est dans la plage d'environ 345 bars à environ 3 500 bars.
